(19) **Europäisches Patentamt**

**European Patent Office**

**Office européen des brevets**

(11) **EP 0 759 941 B1**

(12) **EUROPEAN PATENT SPECIFICATION**

(45) Date of publication and mention
of the grant of the patent:
**13.09.2000 Bulletin 2000/37**

(51) Int. Cl.$^7$: **C07K 14/705**, C07K 14/725,
A61K 39/385, C12N 15/62

(21) Application number: **95918481.3**

(22) Date of filing: **18.05.1995**

(86) International application number:
**PCT/CA95/00293**

(87) International publication number:
**WO 95/31480 (23.11.1995 Gazette 1995/50)**

(54) **HETERODIMER POLYPEPTIDEIMMUNOGEN CARRIER COMPOSITION AND METHOD FOR THEIR USE**

HETERODIMERE TRÄGERZUSAMMENSETZUNG VON IMMUNOGENEN POLYPEPTIDEN UND VERFAHREN ZU DEREN VERWENDUNG

COMPOSITION DE SUPPORT DE POLYPEPTIDE HETERODIMERIQUE IMMUNOGENE ET SON PROCEDE D'UTILISATION

(84) Designated Contracting States:
**AT BE CH DE DK ES FR GB GR IE IT LI LU NL PT SE**

(30) Priority: **18.05.1994 US 245507**

(43) Date of publication of application:
**05.03.1997 Bulletin 1997/10**

(73) Proprietor:
**S.P.I. SYNTHETIC PEPTIDES INCORPORATED
Edmonton, Alberta T6E 5B6 (CA)**

(72) Inventors:
• **HOUSTON, Michael, E.
Edmonton, Alberta T6W 1G4 (CA)**
• **ZHOU, Nian, E.
Edmonton, Alberta T6G 4M5 (CA)**
• **KAY, Cyril, M.
Edmonton, Alberta T5R OP7 (CA)**
• **HODGES, Robert, S.
Edmonton, Alberta T6G 2B2 (CA)**

• **CACHIA, Paul, J.
Edmonton, Alberta T6G OS4 (CA)**
• **IRVIN, Randall, T.
Sherwood Park, Alberta T8H,1E2 (CA)**

(74) Representative:
**VOSSIUS & PARTNER
Siebertstrasse 4
81675 München (DE)**

(56) References cited:
**WO-A-93/15110**

• **PROCEEDINGS OF THE NATIONAL ACADEMY OF SCIENCES OF USA., vol.91, no.24, 22 November 1994, WASHINGTON US pages 11408 - 11412 H.-C. CHIANG ET AL. 'A general method for facilitatin heterodimeric pairing between two proteins; application to expression of alpha and beta T-cell receptor extracellular segments'**

## Description

The present invention relates in general to a composition and methods of use of a polypeptide carrier complex which has two different bioactive moieties attached in a known stoichiometry and molecular orientation. It relates more specifically to a synthetic immunogenic polypeptide complex which presents two different types of antigens in a pre-defined, precise stoichiometry and molecular orientation.

### References Cited

Abbas, A.K., *et al.,* in <u>CELLULAR AND MOLECULAR IMMUNOLOGY,</u> W. B. Saunders Company, Philadelphia PA (1991).
Ausubel, F.M., *et al.,* in <u>CURRENT PROTOCOLS IN MOLECULAR BIOLOGY,</u> John Wiley and Sons, Inc., Media PA.
Benjamin, D.C*., et al*. *Ann. Rev. Immunol*. $\underline{2}$:67 (1984).
Berzofsky, J. A. *Vaccine* $\underline{6}$:89-92 (1988).
Bittle, J.L., *et al*. *Nature (London)* $\underline{298}$:30-33 (1982).
DiMarchi, R., *et al*., *Science* $\underline{232}$:639-641 (1986).
Eisel, U., *et al., EMBO J*. $\underline{5}$:2495-2502 (1986).
Etlinger, H.M., *et al*., *Vaccine* $\underline{9}$:512-514 (1991).
Felix, A.M., *et al*., *Int. J. Peptide Protein Res.* 31:231-238 (1988).
Francis, *et al*., *Nature (London)* $\underline{300}$:168 (1987).
Good, M.F., *et al*. *Ann. Rev. Immunol.* $\underline{6}$:663-688 (1988).
Harlow, *E., at al*., in <u>ANTIBODIES: A LABORATORY MANUAL</u>, Cold Spring Harbor Laboratory Press (1988).
Herzenberg, L.A., *et al*., *J. Exp. Med*. $\underline{155}$:1720-40 (1982).
Ho, P. C., *et al*., *Eur. J. Immunol.* $\underline{20}$:477 (1990).
Hochuli, E. , in <u>GENETIC ENGINEERING, PRINCIPALS AND PRACTICE, VOL. 12</u> (J. Stelow Ed.) Plenum, NY, pp. 87-98 (1990).
Hodges, R.S., *et al*., *Peptide Res*. $\underline{1}$:19-30 (1988).
Hodges, R.S., *et al*., *Peptide Res*. $\underline{3}$:123-137 (1990).
Hodges, R.S., *et al., Peptide Res.* $\underline{3}$:123-137 (1993).
Hodges, R.S., *et al*., U.S. Patent No. 5,223,604, issued June 1993.
Hopp, T.P., *et al*., *Proc. Natl. Acad. Sci. USA* $\underline{78}$:3824-3828 (1981).
Hu, *et al., Science* $\underline{250}$:1400-1403 (1990).
Lerner, R.A. *Nature (London)* $\underline{299}$:592-596 (1982).
McInnes, C., *et al., Biochemistry* $\underline{32}$:13432-40 (1993).
Maniatis, T., *et al*., in <u>MOLECULAR CLONING: A LABORATORY MANUAL,</u> Cold Spring Harbor Laboratory (1982).
Miller, J.F., *et al., Nature (London)* $\underline{216}$:659-63 (1969).
Panina-Bordignon, P., *et al., Eur. J. Immunol* $\underline{19}$:2237-2242 (1989).
Porath, J., *Protein Exp. and Purif.* $\underline{3}$:263 (1992).
Sarin, *et al., Anal. Biochem.* $\underline{117}$:147-157 (1981).
Sela, M. and R. Arnon., in <u>NEW DEVELOPMENTS WITH HUMAN AND VETINARY VACCINES,</u> (Mizrahi, A., *et al.,* Eds.) (Liss, New York), pp 215-323 (1980) .
Skerra, A., *et al*., *Biotechnology* $\underline{9}$ : 273 (1991).
Tam, J.P., *Proc. Natl. Acad. Sci.* USA $\underline{85}$:5409-13 (1988).
Tam, J.P., U.S. Patent No. 5,229,490, issued July 1993.
Wong, W.Y., *et al., Protein Sci.* $\underline{1}$:1308-18 (1992).
Vaccines can be constructed using either largely-intact, native antigenic molecules or portions of antigenic molecules containing the epitope of interest. As discussed by Tam (1988, 1993), recent studies have shown that synthetic peptides can induce antibodies reactive with their respective sequences in the native protein (Sela, *et al*.; Lerner). Antibodies immunoreactive with peptide antigens are useful laboratory and diagnostic reagents. Synthetic peptide antigens, conveniently available through chemical synthesis, can be used for producing immunogens and for passive immunoprophylaxis (Sela, *et al*.; Lerner; Bittle, *et al*.; DiMarchi, *et al*.).

A conventional approach to preparing antibodies immunoreactive with peptide antigens is conjugation of a peptide to a known protein or synthetic polymer carrier to give a macromolecular structure to the immunogenic entity (Sela, *et al*.; Lerner; Bittle, *et al*.). Methods designed to avoid the use of carrier by polymerizing synthetic peptide antigens to give peptide polymers have also been reported (DiMarchi, *et al*.). Although such constructs are effective in producing animal antibodies, they are ambiguous in composition and structure. This is particularly disadvantageous if the antibodies are to be used for a human vaccine.

Vaccines typically comprise an antigen on a natural carrier such as a protein, a carbohydrate, a lipid or a liposome. Such vaccines are useful and have been employed for many years. There are however a number of recognized problems with them, some of which are related to the carrier. Since the carriers are usually isolated from natural sources, they are often not of uniform quality. Additionally, despite expensive and arduous purification efforts, it is difficult, and often impossible, to provide products completely free of natural contaminants. Such contaminants may themselves be antigenic. They cause the undesirable side reactions often associated with the use of vaccines, particularly fevers and tissue swelling. Additionally, the concentration of antigen may vary from one batch to another because the amounts of antigen that react with the carrier or that are observed on its surface are not uniform.

WO 93/15110 discloses chimeric molecules containing bioactive moieties whereby pairs of synthetic peptides pair specifically with one another and preferentially fold as a helical heterodimer.

It is therefore one object of the present invention to provide a polypeptide compound comprised of two subunits that interact to form a coiled-coil heterodimer. Each subunit is derivatized to include a different bioactive moiety which is a T-cell antigen on one subunit and a B-cell antigen on the other subunit and the antigens do not substantially interfere with the formation of a coiled-coil heterodimer. The coiled coil heterodimer may be stabilized by ionic interactions between the subunits.

The antigens may be linked directly to amino acid residues of the carrier polypeptides, or they may be linked through a spacer, such as 2-8 amino acids (*e.g.*, poly-glycine), a carbon chain or the like.

In one embodiment, one subunit is derivatized with a T-cell antigen comprised of a peptide having a sequence represented by SEQ ID NO:10, SEQ ID NO:11, SEQ ID NO:12, SEQ ID NO:13 or SEQ ID NO:14. An exemplary B-cell antigen has the sequence represented as SEQ ID NO:18.

The subunit and its antigen may be a single polypeptide chain, *e.g.*, a fusion polypeptide having two domains which may be separated by a spacer. In one embodiment, the single polypeptide chain has an amino acid sequence that includes a sequence present in SEQ ID NO:28. In another embodiment, the single polypeptide chain has an amino acid sequence that includes a sequence present in SEQ ID NO:30.

It is a related object of the invention to provide a heterodimer polypeptide immunogen comprised of two subunits where each subunit is comprised of a core peptide and an antigen. Each core peptide is comprised of terminal and internal amino acid repeat sequences having the form gabcdef. Positions a and d of each terminal and internal amino acid repeat sequence are isoleucine, leucine or valine, and positions e and g are aspartic acid or glutamic acid in one core peptide, and lysine, arginine or histidine in the other core peptide.

Peptide antigens are attached to the core peptides through covalent linkages to amino acids at position b, c or f of the internal repeats. The two subunits are arranged in a stable α-helical coiled-coil configuration having a 1:1 stoichiometry, and the peptide antigens are disposed toward outer surfaces of the configuration.

The terminal repeat sequences of the each core peptide can include a glutamic acid at position b, a lysine at position f and a lactam bridge formed between positions b and f. The internal repeat sequences can include an amino acid coupling residue at position f, and this coupling residue can be a cysteine residue.

In a preferred embodiment, the core peptides have sequences represented by SEQ ID NO:1 (EE) and SEQ ID NO:2 (KK), and the antigens have sequences represented by SEQ ID NO:12 (T-cell antigen) and SEQ ID NO:18 (B-cell antigen).

It is another object of the invention to provide a pair of subunits for use as an α-helical coiled-coil heterodimer antigen carrier. Each of the subunits contains two terminal amino acid repeat peptide sequences having the form gabcdef, where b is glutamic acid, f is lysine, and b and f are linked by a lactam bridge, and at least one internal amino acid repeat sequence having the form gabcdef, where position b, c or f is a cysteine residue. The cysteine residue can be covalently attached to an antigen. Positions a and d of each terminal and internal amino acid repeat sequence are isoleucine, leucine or valine, positions e and g of one subunit are aspartic acid or glutamic acid, positions e and g of the other subunit are lysine, arginine or histidine.

Two exemplary subunits capable of forming α-helical coiled-coils have sequences represented by SEQ ID NO:1 (EE) and SEQ ID NO:2 (KK).

It is yet another object of the invention to provide a method of preparing a polypeptide compound. The method includes forming two peptide subunits that interact to form a coiled-coil heterodimer. Each subunit is derivatized to include an antigen, each subunit carries a different antigen and the antigens do not substantially interfere with the formation of a coiled-coil heterodimer. The polypeptide subunits are mixed in a benign medium in a ratio of about 1:1 under conditions that promote formation of said coiled-coil heterodimers. The coiled-coil heterodimer may be stabilized by ionic interactions.

One subunit contains a T-cell antigen and the other subunit contains a B-cell antigen. The T-cell antigen can have a sequence represented by SEQ ID NO:10, SEQ ID NO:11, SEQ ID NO:12, SEQ ID NO:13 or SEQ ID NO:14.

It is a related object of the invention to provide a method of preparing a polypeptide immunogen composition, where two core peptides are formed, each of which contains two terminal amino acid repeat sequences having the form gabcdef and at least one internal amino acid repeat sequence having the form gabcdef. Positions a and d of each terminal and internal amino acid repeat sequence are isoleucine, leucine or valine, positions e and g of each terminal and internal amino acid repeat sequence are aspartic acid or glutamic acid in one core peptide, and lysine, arginine or histidine in the other core peptide.

Peptide antigens may be attached through covalent linkages to amino acids at positions b, c or f of the core peptides, and the derivatized peptides are mixed in a benign medium in a ratio of about 1:1 under conditions that promote formation of coiled-coil heterodimers.

A further embodiment of the present invention includes two subunits capable of forming an α-helical coiled-coil heterodimer dimer composition, as described above, where the antigen on the first subunit is replaced by a moiety capable of binding to a target cell, for example, a tumor cell, and the antigen on the second subunit is replaced by a cytotoxic moiety, for example, a radioactive compound.

The first subunit is administered to a subject and allowed to bind to a target cell. Following a selected time interval, the second subunit is administered and allowed to form a heterodimer with the first subunit. The subunits are preferably administered at doses effective to significantly inhibit or kill the target cell while having a minimal cytotoxic effect on non-target cells and causing minimal side-effects in the subject.

These and other objects and features of the invention will be more fully appreciated when the following detailed description of the invention is read in conjunction with the accompanying drawings.

## Brief Description of the Figures

Figures 1a-c show a schematic representation of the synthesis and assembly of an immunogenic formulation disclosed in the specification. Figure 1a shows a schematic of two core polypeptides, each comprised of 5 heptads. Figure 1b shows the core polypeptides after they have been derivatized with antigenic peptides. Figure 1c shows a schematic of an immunogenic complex of the present invention, comprised of two antigen-decorated core polypeptides in a heterodimeric configuration.

Figure 2a shows helical wheel representations of terminal heptads of two exemplary core polypeptides in a parallel α-helical heterodimer configuration. Figure 2b shows helical wheel representations of terminal heptads of two exemplary core polypeptides in an antiparallel α-helical heterodimer configuration.

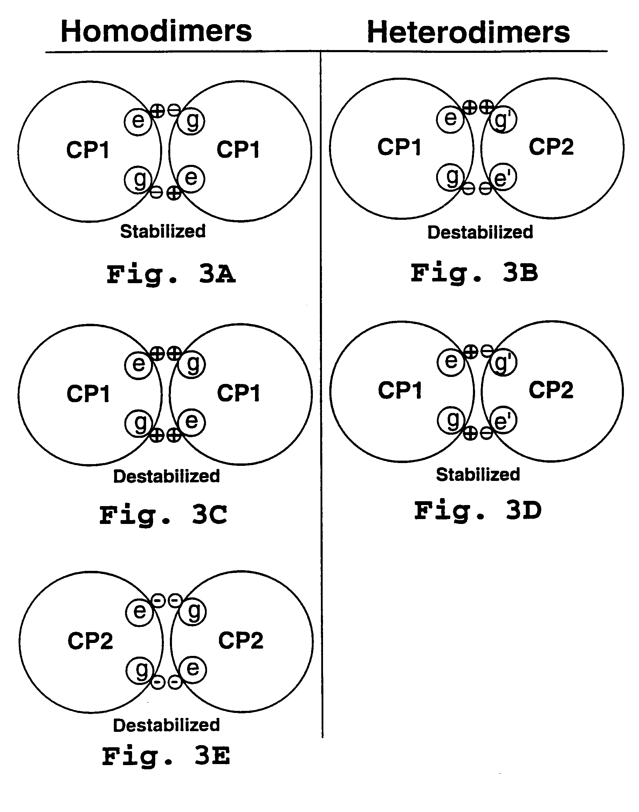

Figures 3a-e show a schematic representations of adjacent heptads of two core polypeptides in a parallel configuration comparing the stabilizing/destabilizing effects of charged residues at the **e** and **g** positions in homodimers vs. heterodimers. Figure 3a shows a homodimer stabilized by oppositely-charged residues at the **e** and **g** positions of a heptad. Figure 3b shows a heterodimer destabilized by oppositely-charged residues at the **e** and **g** positions of a heptad. Figure 3c shows a homodimer destabilized by positively-charged residues at the **e** and **g** positions of a heptad. Figure 3d shows a heterodimer stabilized by like-charged residues at the **e** and **g** positions of a heptad. Figure 3e shows a homodimer destabilized by negatively-charged residues at the **e** and **g** positions of a heptad.

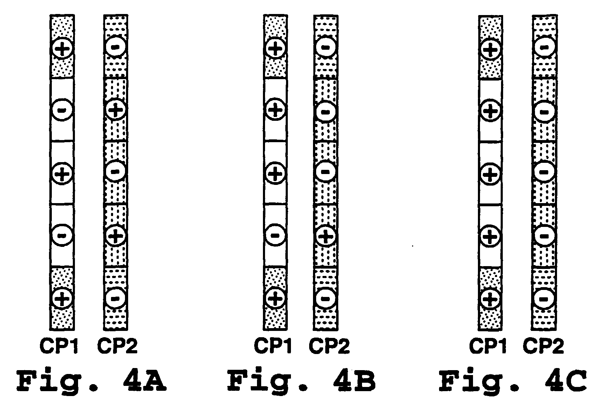

Figures 4a-c show a schematic of some possible distributions of heptads, bearing either positive or negative charges at their **e** and **g** positions, within peptides designed to form coiled-coil heterodimers. Figure 4a shows a schematic of a heterodimer comprised of core polypeptides having alternating positively- and negatively-charged successive heptads. Figure 4b shows a schematic of a heterodimer comprised of core polypeptides, one of which has predominantly positively-charged heptads, and the other of which has predominantly negatively-charged heptads. Figure 4c shows a schematic of a heterodimer comprised of core polypeptides, one of which has all positively-charged heptads, and the other of which has all negatively-charged heptads.

Figure 5 shows a map of plasmid pRLD-E.

Figure 6 shows the polylinker region (promoter, MCS and insert) of plasmid pRLD-E.

Figure 7 shows a map of plasmid pRLD-K.

Figure 8 shows the polylinker region of pRLD-K.

Figure 9 shows a map of plasmid pHIL-S1/E.

Figure 10 shows the polylinker region of plasmid pHIL-S1/E.

Figure 11 shows a map of plasmid pHIL-S1/K.

Figure 12 shows the polylinker region of plasmid pHIL-S1/K.

Figure 13 shows the nucleotide and translated amino acid sequences of a fragment containing sequences encoding PAK pili-C1 cloned in the polylinker region of pHIL-S1/E.

Figure 14 shows the nucleotide and translated amino acid sequences of a fragment containing sequences encoding MVF-C1 (measles virus F protein) cloned in the polylinker region of pHIL-S1/E.

## I. Definitions

The terms "peptide" and "polypeptide", used interchangeably, designate a chain of amino acid based polyamides. The chain can vary in length anywhere from 2 amino acids to 100 or more amino acids. Chains longer than approximately 100 amino acids are typically termed "proteins". Further, the term "heterodimer polypeptide" refers to two associated non-identical polypeptide chains.

The term "derivatized", in the context of a polypeptide subunit "derivatized" to include an antigen, is understood to refer to a polypeptide subunit having one or more antigens covalently attached to one or more amino acid residues forming the subunit, where the antigen may be (i) coupled to one or more amino acid residues in the subunit either before or after polypeptide subunit synthesis, or (ii) form an elongation of the peptide subunit, *e.g.*, at the subunit's N-terminus. Further, the antigen may be attached to the polypeptide subunit directly, or through a linker or spacer, *e.g.*, a poly-glycine spacer.

Unless otherwise indicated, the sequence for peptides and polypeptides is given in the order from the amino terminus to the carboxyl terminus.

The term "epitope" as used herein, designates the structural component of a molecule that is responsible for specific interactions with corresponding antibody (immunoglobulin) molecules elicited by the same or related antigen. More generally, the term refers to a peptide having the same or similar immunoreactive properties, such as specific antibody binding affinity, as the antigenic protein or peptide used to generate the antibody. Therefore, an epitope that is formed by a specific peptide sequence generally refers to any peptide which is reactive with antibodies directed against the specific sequence.

The term "antigen" as used herein, means a molecule which is used to induce production of antibodies. The term is alternatively used to denote a molecule which is reactive with a specific antibody.

The term "B-cell antigen" as used herein, means a molecule which is used to induce production of antibodies. The term is alternatively used to denote a molecule that is reactive with a specific B-lymphocyte clone, or that elicits a B-lymphocyte-mediated immunogenic response in a subject or test animal.

The term "T-cell antigen" as used herein, denotes a molecule that is reactive with a specific T-lymphocyte clone, or a molecule that elicits a T-lymphocyte-mediated immunogenic response in a subject or test animal.

The term "immunogen" as used herein, describes an entity that induces antibody production in a host animal. In some instances the antigen and the immunogen are the same entity, while in other instances the two entities are different.

All amino acid residues identified herein are in the natural or L-configuration unless otherwise specified. In keeping with standard peptide nomenclature, abbreviations for amino acid residues are standard 3-letter and/or 1 letter codes commonly used in the art.

The term "benign medium" as used herein, describes a physiologically-compatible aqueous solution typically having a pH of between about 6 and about 8 and a salt concentration of between about 50 mM and about 500 mM. Preferably, the salt concentration is between about 100 mM and about 200 mM. An exemplary benign medium, designated as buffer A, has the following composition: 50 mM potassium phosphate, 100 mM KCl, pH 7. Equally effective benign media may be made by substituting, for example, sodium phosphate for potassium phosphate and/or NaCl for KCl.

## II. General Overview of the Invention

In one aspect, the invention is a synthetic vaccine formulation having two subunits, each subunit being comprised of a core polypeptide (CP) and one or more antigen molecules (Ag).

The core polypeptides are two non-identical polypeptide chains, typically about 21 to about 70 residues in length, having an amino acid sequence compatible with their formation into two-stranded α-helical heterodimeric coiled-coils in a benign medium. They are designated herein as CP1 (core polypeptide 1), and CP2 (core polypeptide 2). In benign aqueous medium the isolated core polypeptides are random coils. When CP1 and CP2 are mixed together, preferably in equal quantities, they interact to form a two-stranded α-helical heterodimeric coiled-coil carrier, designated as CP1~CP2.

Peptides in an α-helical coiled-coil conformation interact with one another in a characteristic manner that is determined by the primary sequence of each peptide. The tertiary structure of an α-helix is such that 7 amino acid residues in the primary sequence correspond to approximately 2 turns of the α-helix. Accordingly, a primary amino acid sequence giving rise to an α-helical conformation may be broken down into units of 7 residues each, termed heptads. The core polypeptides are comprised of a series of heptads in tandem. When the sequence of a heptad is repeated in a particular core polypeptide, the heptad may be referred to as a "heptad repeat", or simply "repeat".

As is detailed below, specific types of amino acid residues at defined positions in each heptad act to stabilize the two-stranded α-helical coiled-coil heterodimeric structure.

CP1 and CP2 may be independently derivatized, or decorated, with different antigens (Ag1 and Ag2) through amino acid coupling residues. The coupling residues are placed at locations in the sequences of CP1 and CP2 so as to be positioned at the outward, or hydrophilic aspects of an $\alpha$-helical coiled-coil structure. Antigen-derivatized carriers are designated as $[Ag1]_i$-CP1 or CP2-$[Ag2]_j$, where i and j refer to the number of antigens attached to a single core polypeptide. Antigens are selected such that when they are derivatized to core polypeptides, they do not block the formation an $\alpha$-helical heterodimeric coiled-coil structure. $[Ag1]_i$-CP1 and CP2-$[Ag2]_j$ may be purified prior to their assembly into a final immunogenic structure.

CP1 and CP2 may also contain residues that can be reacted (either intra- or inter-helically) to stabilize the $\alpha$-helical or coiled-coil nature of the polypeptides. One example of a stabilizing modification is the incorporation of lactam bridges in the first and last (terminal) repeats of core peptides.

A complete antigenic structure can be made by mixing $[Ag1]_i$-CP1 and CP2-$[Ag2]_j$. The decorated core polypeptides self-assemble to form an antigen-derivatized $\alpha$-helical coiled-coil structure, denoted as $[Ag1]_i$-CP1$\sim$CP2-$[Ag2]_j$. This structure can be used as an immunogen for the production of antibodies or in a vaccine formulation.

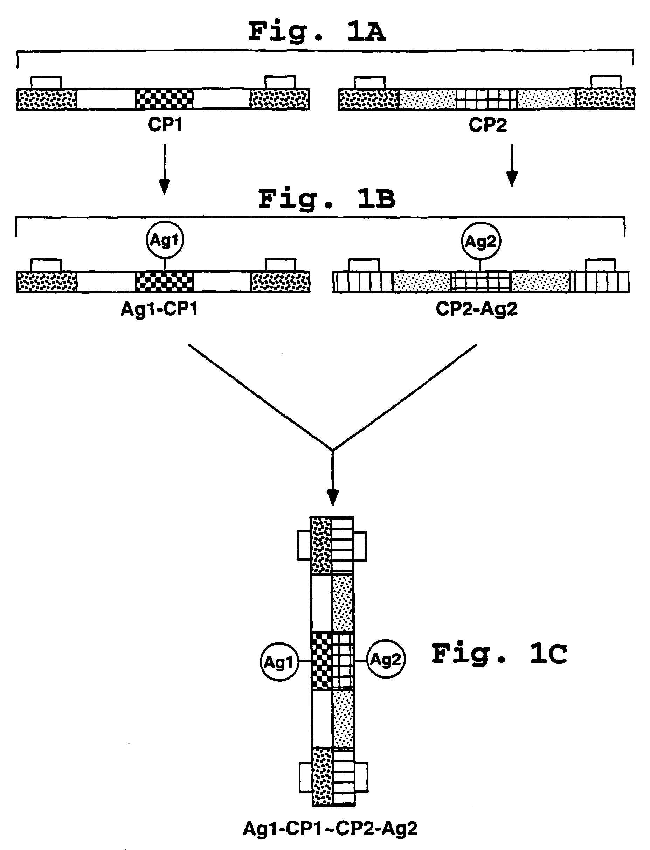

A diagram of the general steps outlined above is shown in Figure 1 for core polypeptides containing 5 heptad repeats (indicated as boxes with varying degrees of shading), one antigen-binding residue per core polypeptide, and lactam-bridge modification sites on the terminal repeats. Part A of Figure 1 shows a schematic of CP1 and CP2 after the peptides had been synthesized under reaction conditions, detailed in Example 4, to induce the formation of lactam bridges. Part B shows a schematic of CP1 and CP2 after the modified core peptides had been derivatized with antigens, as detailed for instance in Example 5. Part C is a schematic of the entire heterodimeric immunogenic complex, shown for simplicity as a linear (as opposed to a coiled-coil) structure, after mixing the individual decorated peptides as described, for instance, in Example 6.

III. Features of Core Polypeptides

The two core polypeptides (CP1 and CP2) are of similar, if not identical size, each typically ranging from about 21 to about 70 residues (3 to 10 heptads) in length.

The peptides may be synthesized by a variety of methods known to those skilled in the art. For example, an ABI Model 420A peptide synthesizer may be used with conventional t-Boc chemistry as described previously by Hodges, *et al*., (1988), and in Example 1.

Subsequent to synthesis, the peptides are purified by any of a number of methods known to those skilled in the art, for example using reversed-phase high performance liquid chromatography (RPC) and a "SYNCHROPAK" RP-P column, as detailed in Example 1.

The composition and purity of the peptides can be verified by several methods, including amino acid composition mass analysis on a Beckman model 6300 amino acid analyzer and molecular weight analysis using time of flight mass spectroscopy on a "BIOION-20" Nordic, as detailed in Example 1.

A. Coiled-Coil Formation

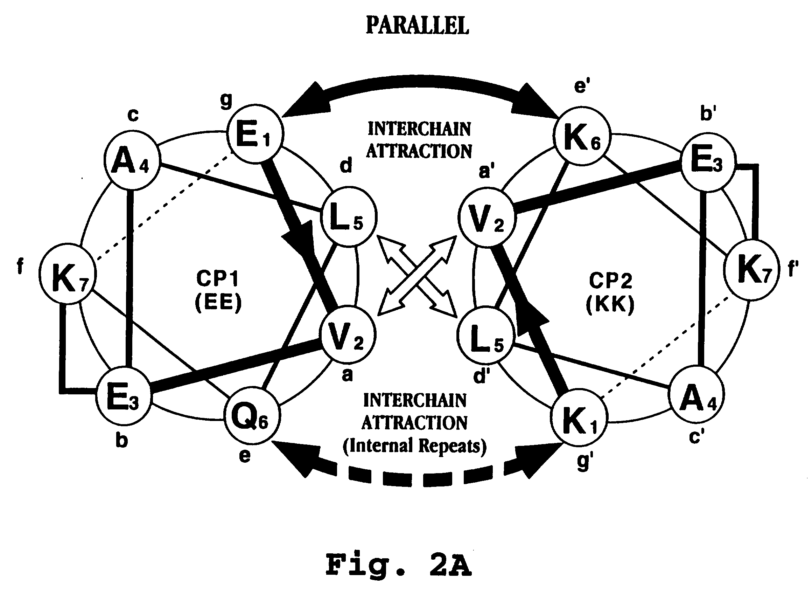

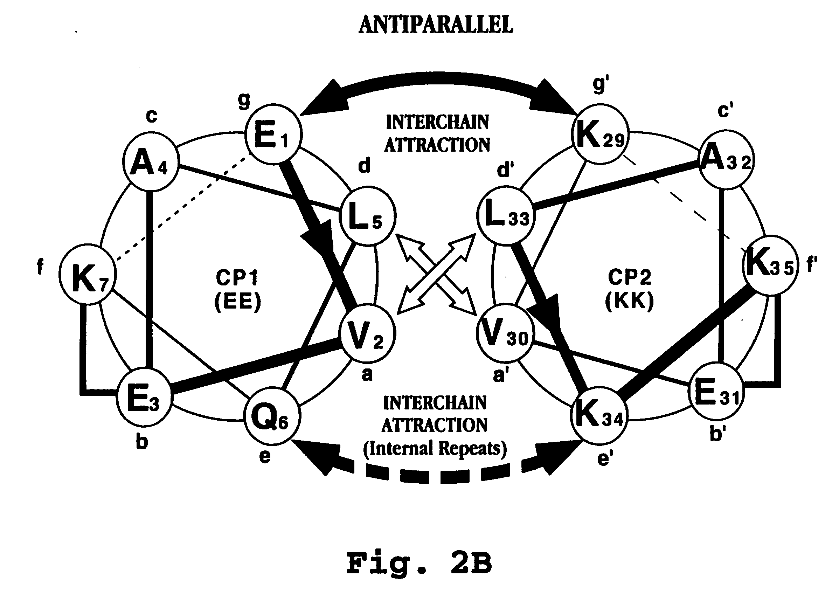

The dimerization of CP1 and CP2 occurs due to the presence of a repeated heptad motif of conserved amino acid residues. The individual positions in each heptad are designated by the letters **a** through **g** for CP1, and **a'** through **g'** for CP2, as shown in Figures 2a and 2b. The positions (*e.g*., **a'**, **g'**) of CP2 are sometimes referred to without the (') symbol in general discussions of heptad positions in core heterodimers, below.

An appropriate heptad motif, or repeat, directs the CP1 and CP2 polypeptides to assemble into a heterodimeric $\alpha$-helical coiled-coil structure under permissible conditions, presented in part D, below. The individual $\alpha$-helical peptides contact one another along their respective hydrophobic faces, defined as the **a** and **d** positions of each heptad.

CP1 and CP2 may assemble into a heterodimer coiled-coil helix (coiled-coil heterodimer) in either parallel or antiparallel configurations. In a parallel configuration, the two core polypeptide helixes are aligned such that they have the same orientation (amino-terminal to carboxyl-terminal). In an antiparallel configuration, the helixes are arranged such that the amino-terminal end of one helix is aligned with the carboxyl-terminal end of the other helix, and *vice versa.*

Diagrams of the relative orientations of the **a-g** positions of two interacting $\alpha$-helices are shown in Figures 2a and 2b. Figure 2a shows an end-on schematic of the first two turns (one heptad) of two exemplary core polypeptides, EE and KK (SEQ ID NO:1 and SEQ ID NO:2) arranged in a parallel configuration. Figure 2b shows an end-on schematic of the same core polypeptides arranged in an antiparallel configuration.

Core polypeptides designed in accord with the guidance presented herein typically show a slight preference for assembling in a parallel orientation *vs*. an antiparallel orientation. Generally, however, the orientation (parallel *vs*. antiparallel) in which the two core polypeptides form an $\alpha$-helical coiled coil is not necessarily relevant to their function as carriers for bringing together moieties attached to the core polypeptides.

In Figures 2a and 2b, amino acids are circled and indicated by the one-letter code, and consecutive amino acid positions are numbered and joined by lines with arrow heads indicating the N-terminal to C-terminal direction. Interactions between the two helixes are indicated by arrows. Wide arrows crossing between the helixes depict hydrophobic interactions between the **a** and **d** positions of adjacent helixes.

Ionic interactions between the **e** and **g** positions of adjacent helixes are indicated as curving arrows above and below the nexus of the helixes. Position **e** of peptide EE (SEQ ID NO:1) is a Gln in the first and last heptad, and a Glu in the internal heptads. The (bottom) curving arrow depicting ionic interactions with this position is drawn with a dashed line to indicate that ionic interactions are present between internal heptads of the helixes, but not between the first and last, or terminal, heptads.

Lactam bridges are indicated as a right-angle line between the **f** and **b** positions within each helix.

B. Hydrophobic Interactions in Coiled-Coil Stability

The hydrophobic interactions between the helixes are due to hydrophobic residues at the **a** and **d** positions of the core polypeptides. Residues at these positions, effective to maintain the helixes in contact, include leucine, isoleucine, valine, phenylalanine, methionine, tryptophan, tyrosine, alanine and derivatives of any of the above. Other residues, including alanine, cysteine, serine, threonine, asparagine and glutamine may also occupy **a** or **d** positions in some heptads, so long as others are occupied by hydrophobic residues.

Appropriate selection of the specific residues to occupy the **a** and **d** positions is an important aspect of the present invention. If the hydrophobic interactions are strong, as is the case, for example, between helixes containing Ile at one of the positions and Leu at the other position, a significant fraction of the helixes will form as homodimers at pH 7, even if like-charged residues are present at the e and g positions to discourage homodimer formation (see part C., below). If, on the other hand, residues at the **a** and **d** positions are selected such that the hydrophobic interactions are too weak (for example, Ala at both positions), the helixes may not form coiled-coil dimers at all. Preferably, residue pairs are selected that promote the formation ≥ 95% heterodimers at pH 7. The degree of heterodimer *vs.* homodimer formation may be measured as described, for instance, in Example 3. An exemplary pair of residues at the **a** and **d** positions, that results in hydrophobic interactions conducive to ≥ 95% heterodimer formation at pH 7, comprises Leu at one of the positions and Val at the other position. These residues are present at the **a** and **d** positions of exemplary core polypeptides EE (SEQ ID NO:1) and KK (SEQ ID NO:2).

C. Ionic Interactions in Coiled-coil Stability

Dimeric coiled-coil conformations of $\alpha$-helixes can be stabilized by ionic interactions between residues at the **e** and **g** positions of adjacent helixes, as is illustrated in Figure 3. If each helix of a dimer has a positively-charged residue at one position, for example, **e**, and a negatively-charged residue at the other position, for example, **g**, homodimer formation is favored (Fig. 3A; compare with heterodimer in Fig. 3B). However, if each helix has like-charged residues at both positions, then two oppositely-charged helixes will tend to associate into heterodimers (Fig. 3D), as opposed to forming homodimers (Fig. 3C, 3E).

The conformation of polypeptides, such as CP1 and CP2, in solution can be determined from CD spectra of the solution. These data provide information as to the conformation of the individual peptides themselves (random coil *vs.* $\alpha$-helical), as well information as to the relative amounts of heterodimer *vs.* homodimer complexes of, for example, CP1 and CP2. Example 2 details one method of measuring CD spectra. Example 3 details how a CD spectra measurements can be used to assess the conformation of peptides in solution.

In the diagram shown in Figure 2, ionic interactions between the two helixes arise from negatively-charged (Glu) residues at the **e** and **g** positions on CP1 (EE; SEQ ID NO:1), and positively-charged (Lys) residues at the **e** and **g** positions on CP2 (KK; SEQ ID NO:2). However, the terminal heptads of peptide EE (SEQ ID NO:1) have uncharged residues (Gln) at the **e** position, as opposed to the charged Glu at that position in internal repeats. Accordingly, ionic interactions involving the **e** position of EE will occur at internal, and not terminal, repeats.

Negatively-charged residues can be aspartic acid, glutamic acid or derivatives thereof. Positively-charged residues can be lysine, arginine, histidine, or derivatives thereof.

Ionic interactions between other positions in a heptad may also exert significant influences on helix stability. For example, position **e** in EE carrier peptide (SEQ ID NO:1) terminal repeats is a Gln, as opposed to a Glu, because Glu residues at both positions would tend to destabilize an $\alpha$-helical conformation through ionic repulsions (see Figs. 2a and 2b). Certain destabilizing effects, however, may be overcome by introducing stabilizing covalent modifications, such as lactam bridge formation discussed below in part E.

D. Conditions Favorable for Coiled-coil Formation

Core polypeptides comprised of repeating heptads and designed according to the guidance presented in parts A through C, above, will readily form coiled-coil heterodimers in a benign medium, defined above in part I. The degree of α-helical coiled-coil heterodimer formation can be determined from CD spectra, as described, for instance, in Example 3.

Coiled-coil heterodimers may form under conditions outside the pH and salt range given for a benign medium, but some of the molecular interactions and relative stability of heterodimers *vs*. homodimers may differ from characteristics detailed above. For example, ionic interactions between the **e** and **g** positions that tend to stabilize heterodimers may break down at low or high pH values due to the protonation of, for example, Glu side chains at acidic pH, or the deprotonation of, for example, Lys side chains at basic pH.

Aforementioned effects of low and high pH values on coiled-coil heterodimer formation may be overcome, however, by increasing salt concentration. Increasing the salt concentration can neutralize the stabilizing ionic attractions or suppress the destabilizing ionic repulsions. Certain salts have greater efficacy at neutralizing the ionic interactions. For example, in the case of the KK peptide (SEQ ID NO:2), a 1M or greater concentration of $ClO_4^-$ anions is required induce maximal α-helical structure (as determined by CD measurements performed as detailed in Example 2), whereas a 3M or greater concentration of $Cl^-$ ions is required for the sane effect. The effects of high salt on coiled-coil formation at low and high pH also show that interhelical ionic attractions are not essential for helix formation, but rather, control whether a coiled-coil tends to form as a heterodimer *vs*. a homodimer.

E. Heptad Variation in Core Polypeptides.

Parts A, B and C, above, present guidelines as to which amino acid residues may be included, and which amino acid residues are preferable, at specific positions in heptads of core polypeptides that will typically result in those peptides forming α-helical coiled-coil structures in a benign medium. This part describes some examples of how heptads with sequences which are in compliance with the guidelines presented in parts A through C, above, can be arranged within the core polypeptides.

Core polypeptides of the present invention may each contain from three to a plurality of heptads. The sequences of each of those heptads may all be the same, or they may differ. In particular, the sequences of the first and last heptads, or terminal repeats, may differ from the sequences of the interior or intermediate heptads or repeats. Furthermore, the sequences of the internal repeats may differ from one another depending on, for example, whether or not the repeats incorporate amino acid coupling residues.

For example, peptide EE (SEQ ID NO:1) has a total of 5 heptad repeats. The two terminal repeats have the sequence represented by SEQ ID NO:3, and the three intermediate repeats have sequences represented by SEQ ID NO:4 and SEQ ID NO:5. The sequence represented by SEQ ID NO:5, present in the central repeat, differs from the internal repeat sequence (SEQ ID NO:4) by the presence of a cysteine coupling residue. Peptide KK (SEQ ID NO:2) also has a total of 5 heptad repeats, and the repeats are arranged in a manner analogous to those of peptide EE. The two terminal repeats of KK have the sequence represented by SEQ ID NO:6, and the three intermediate repeats have sequences represented by SEQ ID NO:7 and SEQ ID NO:8, with the sequence represented by SEQ ID NO:8 including a cysteine coupling residue.

The terminal repeats of both the EE and KK peptides incorporate residues designed to form lactam bridges to stabilize an α-helical conformation. The central internal repeats of both peptides contain amino acid coupling residues (cysteines), and are termed "peptide conjugation internal repeats".

Many other variations in heptad arrangement are possible. For example, it may be desirable to design a core polypeptide with a different amino acid coupling residue on each intermediate repeat, in order to couple different compounds at defined positions on one core polypeptide. This strategy is discussed in more detail in part G, below. Alternatively, one may want to place a unique coupling residue on one of the repeats on one or both core peptides to anchor them to a resin or another polypeptide.

Because the salient interactions between two core polypeptides in an α-helical coiled-coil heterodimer pair are between adjacent, "complimentary" heptads in each peptide, the primary sequence of heptads within a core polypeptide can vary, so long as the residues within each heptad interact favorably with residues in the complimentary heptad of the second core polypeptide.

It follows, then, that adjacent heptads may vary in sequence such that, for example, the net charge on the core polypeptides can be altered without affecting the ability of the polypeptides to form α-helical heterodimer coiled-coils. This relationship is illustrated in Figure 4. The figure shows three examples of CP dimer pairs. Each core polypeptide has 5 heptads. The + or - symbols in each heptad each represent two charges (one at the **e** position and one at the **g** position). Note that adjacent complimentary heptads have opposite charges. For the purpose of this example, it is assumed that positions other than **e** and **g** in each heptad sum to a net charge of zero. It can be appreciated that CP1 and CP2 forming the dimer in Figure 4A have net charges of +2 and -2, respectively, due to an excess of one positively-charged heptad, and one negatively-charged heptad, respectively. Similarly, CP1 and CP2 in Figure 4B have net charges of +6 and -6, respectively, and CP1 and CP2 in Figure 4C have net charges of +10 and -10, respectively. Other variations on this theme are, of course, possible without departing from the spirit of the invention.

Peptides EE (SEQ ID NO:1) and KK (SEQ ID NO:2) are similar to the case schematized in Figure 4C, in that the **e** and **g** positions of all heptads comprising peptide EE have a net negative charge, whereas the **e** and **g** positions of all heptads comprising peptide KK have a net positive charge.

F. Covalent Modification of Core Polypeptides.

The core polypeptide sequences may also include residues designed to stabilize the $\alpha$-helical conformation of each core polypeptide in a coiled-coil dimer. For example, peptides EE and KK have glutamic acid and lysine residues at the **b** and **f** positions, respectively, of the terminal repeats. These residues can react under the appropriate conditions, detailed in Example 4, to form a lactam bridge, as schematized in Figure 1. Lactam bridges at these positions stabilize an $\alpha$-helical conformation.

G. Antigen Coupling to Core Polypeptides

Another aspect of the invention includes the incorporation of amino-acid coupling residues at positions **b**, **c** and/or **f** of one or more heptads. These positions lie along the outward face of a coiled-coil heterodimer. Each heptad may contain up to three coupling residues.

Various embodiments are possible. For example, the amino acid coupling residues may be incorporated in the internal repeat sequences, but not in the terminal repeat sequences. Additionally, coupling residues may be simultaneously incorporated at all three positions, at two of the three positions, or at only one position (for example, **f**) in each heptad. In an exemplary embodiment (EE, KK peptides; SEQ ID NO:1 and 2), the coupling residues are cysteines placed at the **f** position of the central heptad of each core polypeptide.

Preferred coupling groups are the thiol groups of cysteine residues, which are easily modified by standard methods. Example 5 details how the cysteine thiol groups present in the peptide conjugation internal repeats of peptides EE (SEQ ID NO:1) and KK (SEQ ID NO:2) can be used to attach antigenic peptides at those positions. Other useful coupling groups include the thioester of methionine, the imidazolyl group of histidine, the guanidinyl group of arginine, the phenolic group of tyrosine and the indolyl group of tryptophan. These coupling groups can be derivatized in a manner similar to that detailed in Example 5, using reaction conditions known to those skilled in the art.

As was mentioned in part E, above, it may be desirable to incorporate a different amino acid coupling residue in different heptads comprising a core polypeptide, allowing the attachment of different antigens on a single core polypeptide in defined locations. The core polypeptide can be sequentially decorated with the different antigens by carrying out a series of coupling reactions. A single antigen is coupled to the core polypeptide in a given reaction step. In cases where the antigen is a peptide of less than approximately 40 amino acids, it is typically desirable to add a spacer between the antigenic peptide and the core polypeptide. The spacer may comprise, for example, 2 to 5 amino acids. Two exemplary spacers (one for the TT2 peptide, SEQ ID NO:12, and the other for the PAK peptide, SEQ ID NO:18) are detailed in Example 5.

In a preferred embodiment of the invention, the antigens are linked through amino acid spacers to the coupling residues.

In another aspect of the invention, the antigens may be coupled to the core polypeptides not via the amino acid residues at positions **b**, **c** and/or **f**, but rather, directly in or at either end of the core polypeptide (*e.g.*, at the N-terminal or C-terminal end). Such coupling may be carried out using either synthetic or recombinant approaches. In a recombinant approach, polynucleotide sequences encoding the core polypeptides and the antigenic peptides are engineered into suitable expression plasmids using methods known to those skilled in the art (*e.g.*, Maniatis, *et al*., Ausubel, *et al*.). In one embodiment, fusion peptides, containing a core polypeptide or a core polypeptide in tandem with an antigen may then be produced by inducing expression of the plasmids in a suitable expression system and purifying the expressed fusion protein.

The expression plasmid typically contain the following elements: an origin of replication (ori), a selection marker (*e.g.*, ampicillin; Amp-R), a promoter (*e.g.*, lac promoter/operator; lac p/o), a multiple cloning site (MCS) and a transcription terminator. The plasmid may contain a number of other elements, such as signal peptide sequences (*e.g.*, ompA), f1 ori, a flag or affinity sequence to facilitate purification of the recombinant protein (*e.g.*, a His tail) and the like.

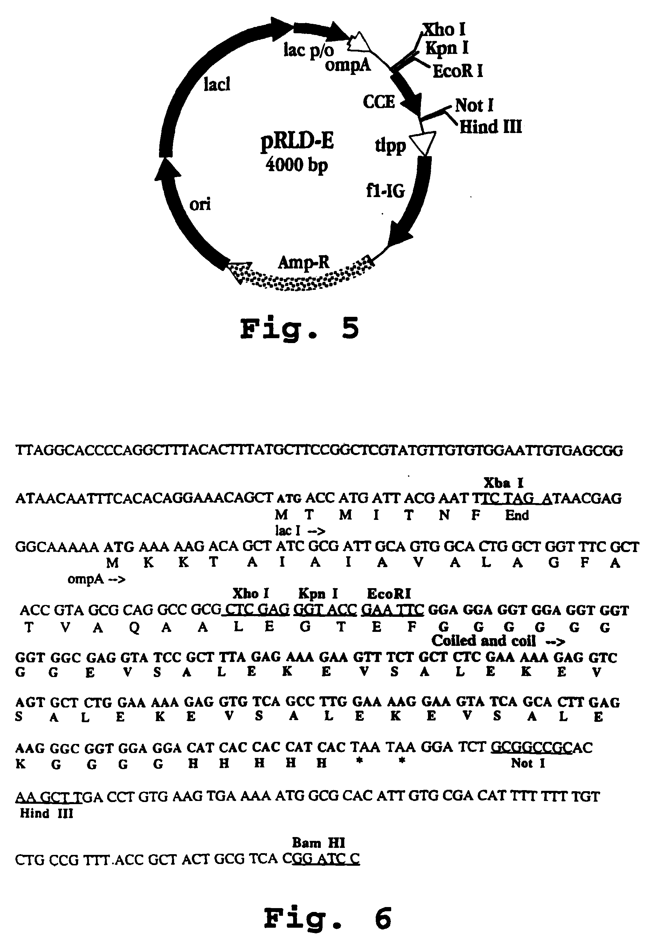

Figures 5-12 illustrate maps (Figs. 5, 7, 9 and 11) and the polylinker regions (Figs. 6, 8, 10 and 12) of four exemplary plasmids suitable for generating recombinant polypeptides useful in the practice of the present invention. Fig. 5 shows a map of plasmid pRLD-E, an *E*. *coli* expression plasmid modified from pASK40 (Skerra, *et al*.) by changing the polylinker sites to correspond to the polylinker of pHIL-S1 and PIC9 (both pHIL-S1 and PIC9 are commercially available from Invitrogen, San Diego,. CA). Fig. 6, illustrating the polylinker region (promoter, MCS and insert) of pRLD-E, shows that pRLD-E contains polynucleotide sequences (SEQ ID NO:19) encoding the E-coil peptide (SEQ ID NO:20), comprised of five repeats of the EE internal repeat (SEQ ID NO:3) in tandem with a 5-residue His tail.

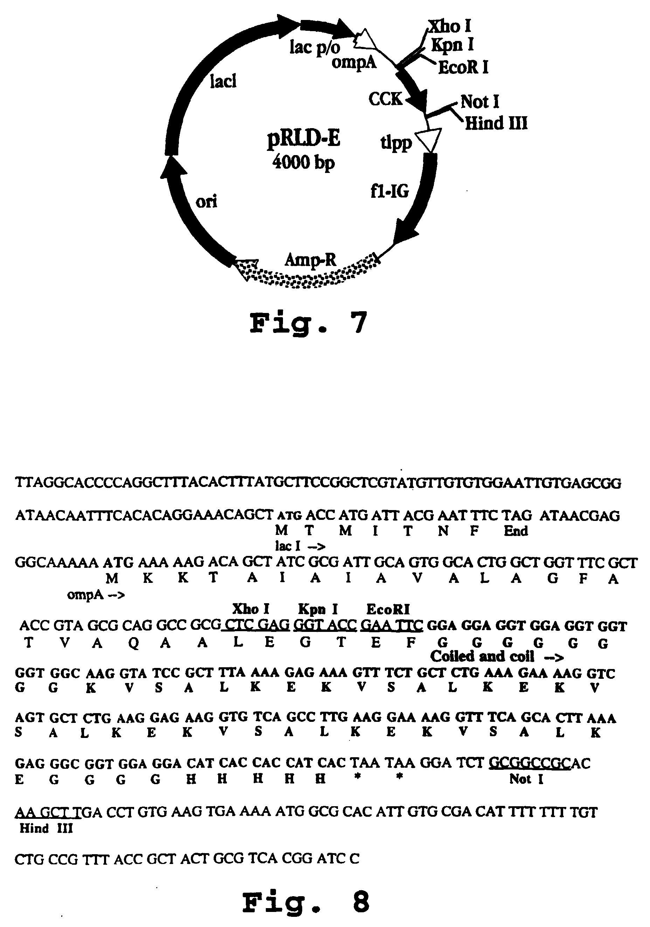

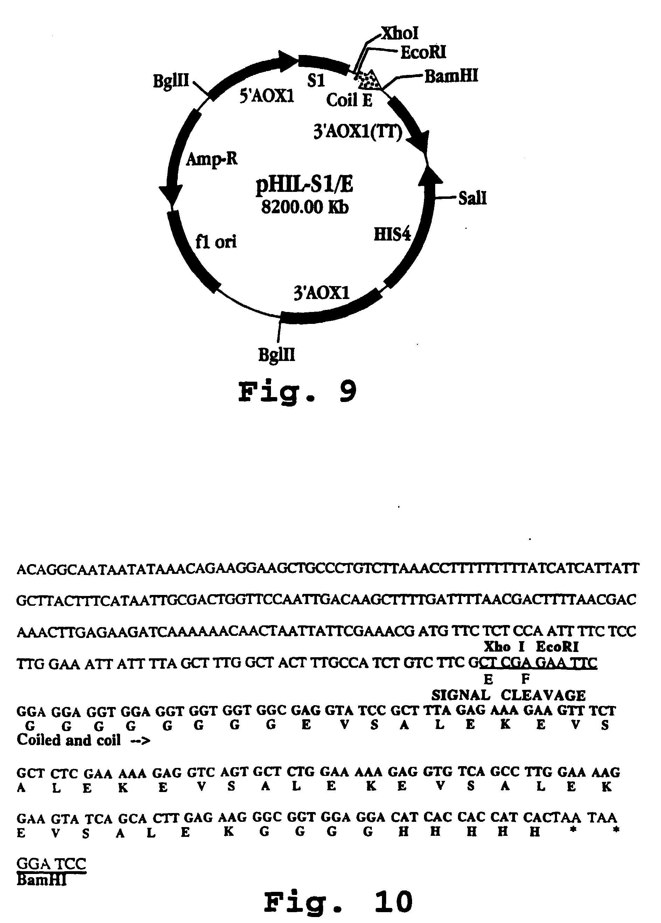

Fig. 7 shows a map of plasmid pRLD-K, which is identical to pRLD-E with the exception that it contains polynucleotide sequences (SEQ ID NO:21) encoding the K-coil peptide (SEQ ID NO:22), comprised of five repeats of the KK internal repeat (SEQ ID NO:7) in tandem with a 5-residue His tail (Fig. 8).

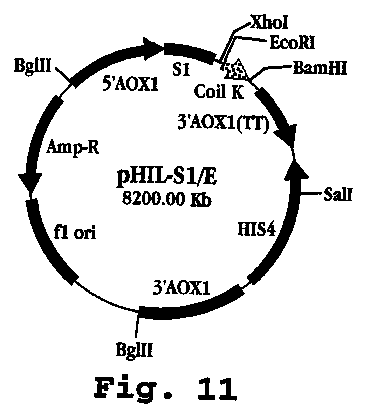

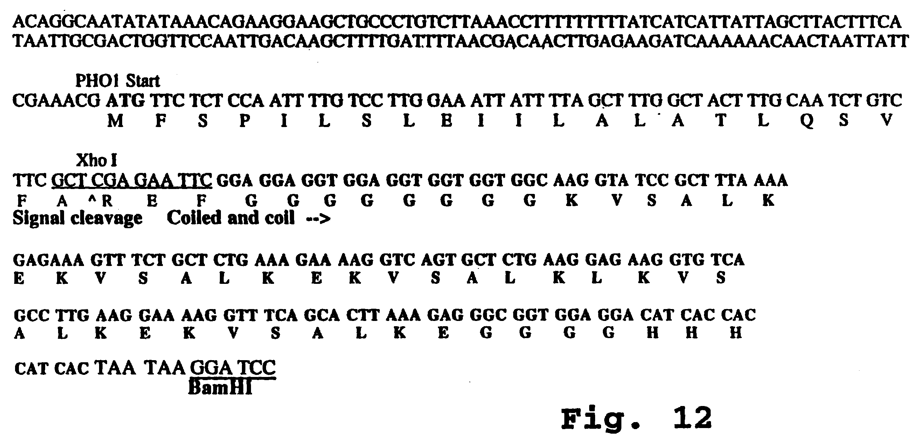

Fig. 9 shows a map of plasmid pHIL-S1/E, a yeast (*e.g., Pichia pastoris*) expression plasmid constructed by cloning an *Eco*RI/*Bam*HI fragment containing a signal cleavage site, a sequence encoding a poly-glycine spacer (8 glycines), a sequence (SEQ ID NO:19) encoding the E-coil peptide (SEQ ID NO:20) another poly-Gly spacer and a His tail (Fig. 10) into the *Eco*RI/*Bgl*II sites of pHIL-S1 (Invitrogen). Fig. 11 shows a map of plasmid pHIL-S1/K, which is identical to pHIL-S1/E with the exception that the insert contains nucleotide sequences (SEQ ID NO:21) encoding the K-coil peptide (SEQ ID NO:22) instead of the E-coil peptide (Fig. 12).

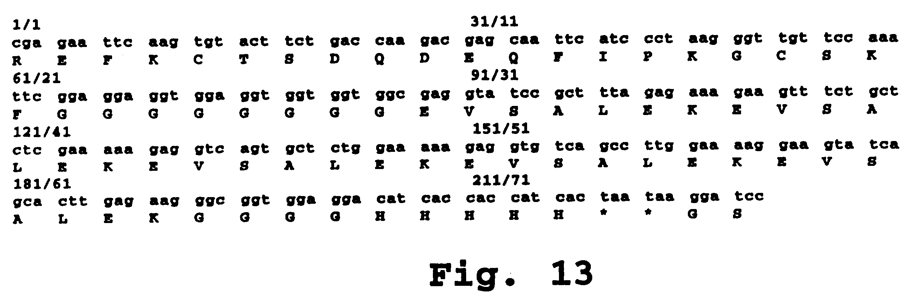

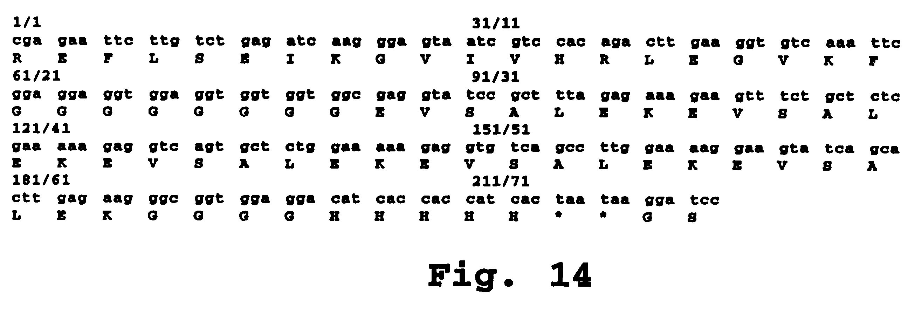

Figs. 13 and 14 show nucleotide and translated amino acid sequence of exemplary fusion construct insert fragments for making recombinant polypeptides suitable for use in vaccine compositions and methods of the present invention. The fragment in Fig. 13 (SEQ ID NO:23) contains a nucleotide sequence (SEQ ID NO:25) encoding a PAK antigen (PAK 128-144; SEQ ID NO:18, SEQ ID NO:26) cloned upstream of the sequence encoding 8 glycines in the polylinker region of pHIL-S1/E. The fusion polypeptide produced from such a fragment, in combination with a corresponding decorated peptide, may be particularly useful as a vaccine composition against *Pseudomonas aeruginosa*, and may be evaluated for such use using, for example, the protocol in Example 8.

The fragment in Fig. 14 (SEQ ID NO:27) contains a nucleotide sequence (SEQ ID NO:29) encoding an MVF T antigen (measles virus F protein; region 288-302; SEQ ID NO:15, SEQ ID NO:30) cloned upstream of the sequence encoding 8 glycines in the polylinker region of pHIL-S1/E.

Expression plasmids such as those described above may be transformed into suitable host cells, such as bacteria or yeast, and induced to produce recombinant polypeptides, which may then be purified using methods known to those skilled in the art and employed for uses such as are detailed herein. Fusion polypeptides containing a poly-His tail, such as those described above, may be conveniently purified by means of immobilized metal ion affinity chromatography (IMAC; Hochuli; Porath).

The pHIL and pPIC -derived vectors are especially suitable for high level expression of recombinant polypeptides. They employ a methanol-regulated alcohol oxidase (AOX) promoter which is particularly useful in *Pichia pastoris* host cells (for example, the AOX promoter is used in pHIL and pPIC vectors included in the *Pichia* expression kit, available from Invitrogen, San Diego, CA). The plasmids are used to transform *Pichia pastoris* (strain GS115; Invitrogen) spheroplasts, and the transformed cells used to produce recombinant polypeptide according to the manufacturer's instructions.

The pRLD-derived vectors may also be employed for expression of recombinant polypeptides of the present invention. The plasmids are used to transform *E. coli* cells (*e.g*., JM83 cells), the cells are induced with isopropyl-β-thiogalactopyranoside (IPTG), the outer membrane is broken, and the periplasmic membrane proteins are isolated and passed over an Ni$^+$ IMAC column (Hochuli; Porath) for purification.

Recombinant proteins purified as described above may be further purified and/or modified using methods known to those skilled in the art (*e.g*., as were used for synthetically-produced peptides described herein) prior to the use of such proteins in the practice of the present invention.

A variety of antigens may be expressed in tandem with a carrier polypeptide, such that they form a single polypeptide chain, to form a decorated peptide. They include antigens, such as exemplified in the constructs shown in Figs. 13 and 14, for use as vaccine compositions.

The specific moieties selected will depend on the application, and can be readily determined by one of ordinary skill in the art following the guidance herein.

Exemplary carrier molecules CP1 and CP2 employed in the recombinant methods described above are the E-coil peptide (SEQ ID NO:20) and the K-coil peptide (SEQ ID NO:22). They differ from EE (SEQ ID NO:1) and KK (SEQ ID NO:) peptides, respectively, in that the E-coil and K-coil peptides are comprised exclusively of "internal" repeats, rather than containing the "terminal" repeats at their ends, but have characteristics (conditions favoring coil-coil formation, etc.) comparable to those of the EE and KK peptides.

H. Generating Antigen-decorated Heterodimers

The individual antigen-decorated core peptides may be purified as detailed in Example 1, precipitated and lyophilized by standard methods. Antigen-decorated heterodimers may be generated by mixing purified [Ag1]$_i$-CP1 with purified CP2-[Ag2]$_j$, as described in Example 6 for the [PAK]$_1$-KK ([PAK]-KK) and EE-[TT2]$_1$ (EE-[TT2]) decorated core polypeptides.

The peptides are individually resuspended in a benign medium, for example buffer A, at a concentration of between about 0.25 mM and 0.5 mM. Approximately equal amounts of each peptide suspended in solution are combined and allowed to react for between 5 and 10 minutes at room temperature. The fraction of peptides in a coiled-coil *vs*. a random orientation is assayed using a CD measurement, as detailed in Example 2. Typically, over 90 % of the total protein is in an α-helical heterodimeric coiled-coil conformation.

Alternatively, equal portions of lyophilized mixtures of the peptides can be mixed and resuspended in benign medium.

IV. Advantages for Vaccine Development

Important features of the present invention related to vaccine development include (i) two or more different types of antigens, comprised of a plurality of individual antigenic polypeptides, can be incorporated into one immunogenic macromolecule of well-defined structure, (ii) the components are synthesized and purified to homogeneity prior to their assembly, allowing for control over the composition at each step of synthesis, and enabling the production of a pure, well-defined product and (iii) a high concentration of antigens can be achieved in a relatively small volume.

These features are advantageous in the design of effective and reproducible vaccines.

An effective vaccine must elicit a strong immune response. To elicit an immune response that affords potent and prolonged protection, it is desirable to stimulate both B- and T-lymphocytes (B- and T-cells; Benjamin, *et al*.). B-cells respond to circulating antigens that bind to specific immunoglobulin (Ig) receptors on their surface, whereas T-cells are stimulated by binding to antigens that had been internalized, processed and appropriately presented by antigen presenting cells (APCs). APCs present foreign antigens on their surface as antigen fragments bound to the major histocompatibility complex (MHC), for recognition by T-cells bearing the appropriate T-cell receptor complex (Abbas, *et al*.).

B-cell and T-cell epitopes are typically not identical, even though both may be derived from the same immunogenic molecule (Benjamin, *et al*.). Effective T-cell antigens are usually amphipathic helixes, presumably because the hydrophobic face interacts well with a groove in the MHC type II and the hydrophilic face is exposed to the extracellular medium for interaction with the T-cell receptor (Berzofsky).

The strongest immune responses are mounted when a B-cell functions as an APC. This brings B- and T-cells in close proximity and increases the effectiveness of cytokines, released by both cell types, that stimulate the cells to proliferate and generate "memory" cells.

A B-cell displaying the appropriate Ig antibody binds a B-cell antigen on a foreign antigenic molecule, internalizes the molecule, processes it, and displays a T-antigen fragment in association with an MHC type II for binding by an appropriate helper T-cell.

Native antigen molecules typically contain both B-and T-cell antigens, and are thus capable of eliciting a strong immune response. There are several disadvantages, however, to using intact proteins as antigens, particularly in human vaccines. They include (i) the chance of generating antibodies against a part of an antigenic molecule that is variable among closely-related strains of the pathogen (thus reducing the effectiveness of the vaccine), and (ii) the chance of generating antibodies to an epitope that is similar to one in an endogenous protein, thus increasing the risk of developing an autoimmune response. Furthermore, obtaining intact protein in large quantities and of sufficient purity for use in humans is difficult. The purification of crude antigens isolated from the pathogenic organism is tedious, costly and carries with it the risk of infection for individuals involved in production. Large-scale culture of bacteria or yeast to harvest and purify recombinant proteins in the amounts required for vaccination of a large number of individuals is impractical (Good).

The present invention offers an alternate to the use of intact antigenic molecules for eliciting a strong immune response. According to one method of the invention, a synthetic polypeptide comprising, for example, a B-cell epitope is derivatized to CP1, and a synthetic polypeptide bearing, for example, a T-cell epitope is derivatized to CP2. The decorated polypeptides are purified and mixed to form a stable heterodimer coiled-coil structure decorated on its outer surface by epitopes of interest.

A vaccine formulation made according to the present invention may thus incorporate well-characterized, effective B-cell antigen peptides together with proven and effective T-cell antigen peptides coupled to a single molecule. Such a formulation is highly reproducible because the antigens are present in a pre-defined molecular orientation and stoichiometry that is essentially invariant from batch to batch.

Among the advantages of the present invention are that the exact structure is known, there are no contaminants which may themselves be antigenic, produce tissue irritation, or other undesirable reactions, the exact amount and orientation of the antigen is known, the antigen is symmetrically-distributed on the carrier, the components can be purified independently to homogeneity prior to final assembly and the carrier can be utilized as a base for more than one antigen, so that multivalent vaccines can be produced. Unlike previous systems using natural carriers such as keyhole limpet hemocyanin, tetanus toxoid and bovine serum albumin, the carriers of this invention are fully-defined chemical entities to which the antigens are derivatized in known orientations and stoichiometries.

The present invention addresses the above-identified shortcomings of current immunogenic formulations and vaccines, and furthermore provides a general method of assembling and presenting two different bioactive moieties in a well-defined spacial orientation and stoichiometry.

V. Selection of Peptide Antigens

In a preferred embodiment of the invention, the substances linked to the core molecules are antigenic peptides, for the construction of antigenic formulations to be used in antibody production or vaccine development. In an exemplary embodiment, the invention includes a B-cell antigen linked to one core polypeptide (e.g., CP1), and a T-cell antigen linked to the other core polypeptide (e.g., CP2).

A. B-cell Antigens

Effective vaccines result in the production of antibodies by B-cells in the vaccinated individual which are directed against epitopes of the pathogen. Some epitopes are more antigenic than others, and readily stimulate the production of potent antibodies effective to inactivate the pathogen. The identification of particularly antigenic B-cell epitopes, and epitopes that will significantly inhibit the pathogen, depends on the resources available. The techniques for such an identification, however, are well-known to those skilled in the art. Several examples are listed below.

If the DNA encoding a particularly antigenic protein of the pathogen has been cloned, it may be possible to use one of a number of computer programs to identify regions of isolated sequences that are likely to encode protein antigenic determinants (for example, Hopp, et al.; "ANTIGEN," Intelligenetics, Mountain View CA).

If sera from infected individuals are available, one can screen the sera, either individually or in a mixture, using an ELISA assay such as is described in the Materials and Methods section of the present invention, to identify reactive proteins or peptides.

If an animal model for a disease or affliction exists, one can screen antibodies generated against defined proteins or peptides of the pathogen for the antibodies' ability to neutralize the infectivity of a virulent mixture of pathogen administered to the model animal.

Effective antigens may also be identified as regions of pathogen proteins that are involved in specific host-pathogen interactions in the disease cycle. This is true particularly in cases where a cellular model for the disease or affliction exists, as in the case for example, for Pseudomonas aeruginosa infection. As demonstrated by Hodges, at al. (1993), peptides derived from exoenzyme S (Exo S), a bacterial toxin having ADP ribosyl transferase activity which is present on the surface of P. aeruginosa cells, and antibodies directed against these peptides, are effective to block the attachment of P. aeruginosa and other micro-organisms to tracheal epithelial cells (TECs) and buccal epithelial (BECs). The Exo S peptide antigen includes the sequence represented by SEQ ID NO:9.

Other examples of peptides that are effective B-cell antigens and that can be used in vaccine formulations designed to protect against the respective organisms include the MVF peptide from measles F protein (residues 288-302; SEQ ID NO:15; SEQ ID NO:30), the HBV peptide (a hepatitis T antigen; SEQ ID NO:16), the CSP peptide from P. vivax CSP protein, residues 317-336 (SEQ ID NO:17), and the PAK Peptide (P. aeruginosa strain K pilin antigen, residues 128-144; SEQ ID NO:18, SEQ ID NO:26).

Although some B-lymphocytes have been found to interact directly with certain antigens, the majority of B-cells, and all memory B cells, have been found to require cooperation with T-cells before they can differentiate towards antibody secretion. A brief summary of T-cells and T-cell antigens, as it relates to aspects of the present invention, is presented below.

B. T-cell Antigens

In many cases, pathogen epitopes that, due to their accessibility, structural invariance among different pathogenic strains, or unique role in the life cycle of the pathogen, would be well-suited for targeting by a vaccine, are not particularly antigenic. The antigenicity of these epitopes can be increased by coupling them to a highly immunogenic carrier protein, such as tetanus toxoid. Unfortunately, this strategy has not been uniformly successful in clinical trials (Etlinger, et al.). One reason may be that the carrier proteins were themselves used in previous vaccinations of the individuals, either as carriers for other vaccines or as vaccines themselves (e.g. tetanus toxoid), and have resulted in epitopic suppression. Epitopic suppression occurs when pre-immunization with a carrier protein can inhibit the subsequent antibody response to new epitopes attached to the carrier protein (Herzenberg, et al.). By using peptides derived from antigenic carrier proteins, as opposed to the intact carrier proteins, epitopic suppression can be made advantageous. It appears that certain such peptides, termed "helper" peptides (Francis, et al.), are recognized by previously-primed helper T-cells, but not by cells responsible for suppression (B-cells and suppressor T-cells).

Some helper peptides, in combination with B-antigens, elicit immune responses that are genetically restricted to only one or a few alleles of class II MHC. This phenomenon of MHC "restriction" arises from the fact that T-cells do not recognize the native protein, but a processed form of protein antigen. The resulting fragments must presented on the surface of cells bearing the same haplotype as the T-cells themselves, but not on cells bearing different haplotypes. Recent data show that some T-antigenic peptides are permissive in their interaction with a wide range of MHC haplotypes (Ho, et *al*.). In particular, peptides derived from tetanus toxoid are typically very effective at stimulating T-cells.

These peptides include the TT0 peptide (tetanus toxoid residues 88-99; SEQ ID NO:10), TT Peptide (also referred to as TT12, tetanus toxoid residues 580-599; SEQ ID NO:11), TT2 peptide (also referred to as P2; tetanus toxoid residues 830-846; SEQ ID NO:12), TT1 peptide (also referred to as TT21; tetanus toxoid residues 916-932; SEQ ID NO:13), and TT3 peptide (also referred to as P30; tetanus toxoid residues 947-967; SEQ ID NO:14).

According to a method of the present invention, an effective vaccine formulation can be constructed utilizing an appropriate B-cell antigen in combination with an antigenic T-cell antigen capable of interacting with a wide range of MHC haplotypes. One such exemplary formulation is identified in section VI, below.

VI. Exemplary Carrier/antigen Combination

An exemplary vaccine composition of the present invention contains the PAK peptide (SEQ ID NO:18; B-cell antigen) coupled to the KK (SEQ ID NO:2) core peptide, and a tetanus toxoid peptide (TT2, SEQ ID NO:12; T-cell antigen) coupled to the EE (SEQ ID NO:1) core peptide.

The PAK peptide (SEQ ID NO:18) has been previously identified as an effective B-cell antigen (Wong, et *al*., 1992). The epitope formed by this peptide is recognized by *Pseudomonas aeruginosa* strain K-specific monoclonal antibody PK99H, which blocks pilus-mediated adherence to buccal and tracheal epithelial cells (Wong, et *al*, 1992).

The TT2 peptide (SEQ ID NO:12) was chosen as a T-cell antigen based on work by Panina-Bordignon, *et al*. (1989). These authors showed that the TT2 peptide, as well as the TT3 peptide (SEQ ID NO:14), are universally immunogenic, since they are recognized by all primed (human) donors, irrespective of their MHC haplotypes.

The EE (SEQ ID NO:1) and KK (SEQ ID NO:2) core peptides are exemplary CP1 and CP2 core polypeptides. Both peptides contain Val residues at their **a** positions, and Leu residues at their **d** positions, ensuring hydrophobic interactions effective to stabilize coiled-coil heterodimers, but not strong enough to overcome the electrostatic repulsion between homodimers.

The **e** and **g** positions of EE internal repeats (SEQ ID NO:4, SEQ ID NO:5) contain Glu residues, whereas the **e** and **g** positions of both terminal (SEQ ID NO:6) and internal repeats (SEQ ID NO:7, SEQ ID NO:8) of KK contain Lys residues. The opposite charges at corresponding positions within complimentary heptads of EE and KR stabilize α-helical coiled-coil heterodimers, as was described in section III, parts C and D above, and illustrated in Figs. 3a-e and 4.

In an analogous manner, the charged groups at the **e** and **g** positions discourage the formation of, and destabilize homodimers. According to an aspect of the present invention, this destabilization is strong enough to overcome the hydrophobic interactions present between appropriately-chosen residues at the **a** and **d** positions, which favor the formation of both heterodimers and homodimers.

The terminal repeats of both peptides contain Glu at the **b** positions, and Lye at the **f** positions, which can form intra-helical lactam bridges. The lactam bridges can be formed, for instance, under the reaction conditions detailed in Example 4. The bridges, schematized in Figures 2a and 2b as straight lines forming a right angle and connecting positions **b** and **f** within each α-helix, stabilize an α-helical conformation when formed under the appropriate conditions, detailed in Example 4.

The peptide conjugation internal repeats of both peptides (SEQ ID NO:5 (EE) and SEQ ID NO:8 (KK)) contain Cys at the **f** position. The thiol groups of these cysteines are used to couple antigenic peptides to the core polypeptides using, for instance, the protocol detailed in Example 5. A B-cell antigenic peptide, the PAK strain pilin antigen peptide (SEQ ID NO:18) is coupled to the Cys residues of the internal repeats in the KK peptide (SEQ ID NO:2), while the tetanus toxoid derived TT2 peptide (SEQ ID NO:12) is coupled to the Cys residues of the internal repeats in the EE peptide (SEQ ID NO:1).

Another set of exemplary vaccines includes the recombinantly-produced fusion peptides described above. For example, the polypeptide encoded as shown in Fig. 13, contains the PAK antigen coupled to the E-coil carrier peptide. The fusion protein may be expressed and purified as described above, and used as an antigen-decorated core peptide (as described above) in conjunction with a complimentary (*e.g.*, K-coil or KK-based) antigen-decorated (*e.g.*, T-antigen) core peptide to make a vaccine composition. The conditions for mixing the decorated core peptides are as were used above.

VII. Antibodies and Immunizations

A. Antibodies

In another aspect, the invention includes the production of specific antibodies directed against polypeptide formulations of the present invention. To prepare antibodies, a host animal, such as a rabbit, is immunized with a polypeptide formulation of the present invention. The host serum or plasma is collected following an appropriate time interval, and this serum is tested for antibodies specific against the antigen. The gamma globulin fraction of the IgG antibodies of immunized animals can be obtained, for example, by use of saturated ammonium sulfate or DEAE "SEPHADEX", or other techniques known to those skilled in the art for producing polyclonal antibodies.

Alternatively, an antigenic formulation of the present invention may be used for producing monoclonal antibodies. Here the spleen or lymphocytes from an immunized animal are removed and immortalized or used to prepare hybridomas by methods known to those skilled in the art.

Example 7 describes the production of mouse antibodies which are specific against the PAK antigenic peptide (SEQ ID NO:18) in the [PAK]-KK~EE-[TT2] synthetic vaccine formulation.

B. Vaccines and Neutralizing Antibodies.

Vaccines can be prepared using immunogenic polypeptides synthesized by the method of the present invention. One way to identify potential antigens which may be useful as vaccines is by screening for antigens which result in neutralizing antibodies. The protocols for achieving this are well-known in the art. Briefly, a potentially-antigenic formulation is used to prepare antibodies in a suitable animal, for example a rabbit. Antibodies or antibody-containing serum are then isolated from the animal and incubated with a virulent mixture of the pathogen against which the antibodies were designed. The pathogenicity of the mixture is then evaluated in an appropriate assay system, for example a model animal or susceptible cell culture, and compared with the (positive control) pathogenicity of a pathogenic mixture incubated only with adjuvant or carrier. Neutralizing antibodies will significantly diminish the infective potential of the pathogenic mixture. An antigenic polypeptide that produces good neutralizing antibodies is considered to be an effective immunogenic polypeptide.

Vaccines containing immunogenic polypeptides as active ingredients are typically prepared as injectable either as solutions or suspensions. Further, the immunogenic polypeptides may be prepared in a solid or lyophilized state that is suitable for resuspension, prior to injection, in an aqueous form. The immunogenic polypeptides may also be emulsified or encapsulated in liposomes. The polypeptides are frequently mixed with pharmaceutically acceptable excipients that are compatible with the polypeptides. Such excipients include, but are not limited to, the following and combinations of the following: saline, water, sugars (such as dextrose and sorbitol), glycerol, alcohols (such as ethanol), and others known in the art. Further, vaccine preparations may contain minor amounts of other auxiliary substances such as wetting agents, emulsifying agents (*e.g*., detergents), and pH buffering agents. In addition, a number of adjuvants are available which may enhance the effectiveness of vaccine preparations. Examples of such adjuvants include, but are not limited to, the following: the group of related compounds including N-acetyl-muranyl-L-threonyl-D-isoglutamine and N-acetyl-nor-muranyl-L-alanyl-D-isoglutamine, and aluminum hydroxide.

The polypeptides are commonly formulated into vaccines in neutral or salt forms. Pharmaceutically acceptable organic and inorganic salts are well known in the art.

Other possible formulations include oral and suppository formulations. Oral formulations commonly employ excipients (*e.g*., pharmaceutical grade sugars, saccharine, cellulose, and the like) and usually contain within 10-98% immunogenic polypeptide. Oral compositions take the form of pills, capsules, tablets, solutions, suspensions, powders, etc., and may be formulated to allow sustained or long-term release. Suppository formulations use traditional binders and carriers and typically contain between 0.1% and 10% of the immunogenic polypeptide.

An example of a vaccine is a composition including the [PAK]-KK~EE-[TT2] polypeptide immunogen described in section VI, above. This immunogen is used as a vaccine against infection by microorganisms which have surface proteins which are antigenically cross-reactive with antibodies produced against the epitope formed by the sequence SEQ ID NO:18, as described in Example 8.

In view of the above information, multivalent vaccines against a variety of antigens can be generated.

The vaccines of the present invention are administered in dosages compatible with the method of formulation, and in such amounts that will be pharmacologically effective for prophylactic or therapeutic treatments. The quantity of immunogen administered depends on the subject being treated, the capacity of the treatment subject's immune system for antibody synthesis, and the desired level of protection. The amounts to be administered are routinely determined by the administering health care professional.

The vaccines of the present invention can be administered in single or multiple doses. Dosage regimens are also determined relative to the treatment subject's needs and tolerances.

VIII. Utility

Compositions made according to the methods of the present invention can be used in a number of ways. Several examples are described below.

A polypeptide designed according to one aspect of the present invention can be used as a general immunocarrier, derivatized with antigenic substances or polypeptides of choice. The general immunocarrier can be used to produce antibodies in rabbits, or antibodies in mice using well-known methodologies. Further, the immunocarrier can be used in vaccine formulations.

As an example, a general immunocarrier can be synthesized with an antigen that is cross-reactive with antibodies effective to inhibit *P. aeruginosa* infection in animals. In this embodiment of the invention, one subunit would be derivatized with a B-cell antigen, such as PAK peptide (SEQ ID NO:18), and the other subunit would be derivatized with a T-cell antigen, such as a tetanus toxoid peptide TT2 (SEQ ID NO:12). An immunocarrier designed in this manner can be used as part of a vaccine formulation to protect against *P. aeruginosa* in animals. In a related embodiment of the invention, an immunocarrier can be designed with an antigen that is cross-reactive with antibodies effective to inhibit a *P. aeruginosa* infection in humans, and can be used as part of a vaccine formulation to protect against *P. aeruginosa* infection, or to ameliorate an existing *P. aeruginosa* infection in humans.

Compositions synthesized according to the present invention can also be used as part of a vaccine and/or antibody development kit. Core polypeptides included in such a kit can be sold with the coupling residues already activated, such that all that is required to generate $[Ag1]_i$-CP1 and CP2-$[Ag2]_j$ is the addition of activated CP1 to a solution containing Ag1 and the addition of activated CP2 to a solution containing Ag2.

Alternatively, the kit can be sold with non-activated core polypeptides, with appropriate instructions for carrying out coupling reactions, and (optionally) including required coupling reagents. A kit formulated in this manner can also include an exemplary T-cell helper peptide capable of interacting with a wide range of MHC haplotypes. The T-cell helper peptide can be already coupled to one of the core polypeptides, or it can be included as a separate reagent. The later case provides the option of using a T-cell antigen of the user's own choosing.

Compositions made according to one aspect of the present invention can be used to develop potentially therapeutic antibodies. Antibodies can be developed, for example, by (i) following the guidance set forth in the present specification, or (ii) using a kit developed in accordance with the present invention, as described in the above paragraph. Therapeutic antibodies can be produced in any appropriate animal by techniques well known in the art, for example the methods detailed in Example 7. Such antibodies can be used to treat diseases or afflictions for which they were developed, or for diagnosing such diseases and afflictions.

Polypeptides designed according to an aspect of the present invention can also be used as a "molecular glue", that can bring together two different bioactive moieties linked to CP1 and CP2. This strategy can find applications *in vivo*, both intra-cellularly and extracellularly, as well as *in vitro*, in cell-free extracts, homogenates, or general reaction mixtures where it is desired to bring into close apposition two polypeptides or other substances.

A "molecular glue" application, such as is presented in the above paragraph, can be made largely irreversible, by the incorporation of inter-helical coupling residues, or by utilizing conditions under which the decorated core polypeptides remain almost exclusively as α-helical heterodimeric coiled-coils.

Alternatively, the "molecular glue" could be made reversible. For example, core polypeptides can be designed that will associate into α-helical coiled-coil dimers under one set of reaction conditions, but dissociate into monomers under a different set of conditions. The different conditions can include changes in variables such as pH and salt concentration, the effects of which on coiled-coil formation are outlined in section III, part D. Conditions compatible with a selected "molecular glue" application that enable reversible coiled-coil formation in a selected application can be determined based on the guidance in the specification.

The following examples illustrate, but in no way are intended to limit the present invention.

Materials and Methods

Overview of ELISA Protocol

A purified antigenic polypeptide formulation is immobilized on a solid support, such as a multiwell polystyrene plate. Sera to be tested are diluted and added to the wells. After a period of time sufficient for the binding of antibodies to the immobilized antigens, the sera are washed out of the wells. A labelled reporter antibody is added to each well along with an appropriate substrate: wells containing antibodies bound to the immobilized antigen polypeptide are detected by a positive signal.

15

ELISA Protocol

(adapted from Worobec, E.A., *et al., J. Biol. chem.* 260:938 (1985).)

Antigenic peptides (10 mg/Ml in 0.01 M carbonate buffer, pH 9.5) are added to each well (100 µl/well) of a NUNC 96-well polystyrene plate and left for 6 hours at room temperature. The wells are washed 3 times with 250 µl of PBS pH 7.4 supplemented with 0.02% (wt/vol) BSA (wash buffer), and 250 µl 5% (wt/vol) BSA in PBS pH 7.4 are added to each well. The plates are incubated overnight at 4°C to block non-specific binding sites in the wells. The wells are then washed three times with wash buffer and 100 µl of primary mouse antibody is added and allowed to incubate at room temperature for 2 hours. The wells are washed 3 times with 250 µl wash buffer. A goat anti-mouse IgG (H+L) immunoglobulin-horse radish peroxidase conjugate (Jackson Laboratories, Bar Harbor, ME) in wash buffer is added (100 µl/well) and incubated for 2 hours at room temperature. The wells are washed 3 times with wash buffer and 350 µl of substrate solution are added to each well. The substrate solution consists of 1 mM 2,2'-azino-di-(3-ethylbenzthiazoline sulfonic acid), 0.03% (vol/vol) hydrogen peroxide in 10 mM sodium citrate buffer at pH 4.2. The reaction is stopped by the addition of 250 µl/well of 4 mM sodium azide. Absorbance at 405 nm is determined using an EL-407 plate reader.

Example 1

Peptide Synthesis, Purification and Analysis

All peptides were synthesized by solid-phase peptide synthesis using a benzhydryl amine-hydrochloride resin on an Applied Biosystems (Foster City, CA) peptide synthesizer Model 430A with conventional *N-t*-butyloxycarbonyl (*t*-Boc) chemistry as described previously (Hodges, *et al.*, 1988). The peptides were cleaved from the resin by reaction with hydrofluoric acid (HF; 20 ml/g resin) containing 10% anisole and 2% 1,2-ethanedithiol for 1 hour at -5°C to 0°C.

The crude reduced peptides were purified by reversed-phase high performance liquid chromatography (RPC) and a "SYNCHROPAK" RP-P semi-preparative $C_{18}$ column (250 × 10 mm inner diameter, 6.5 µm particle size, 300 Å pore size; SynChrom, Lafayette, IN) with a linear AB gradient of 0.5% B/min and 2 ml/min, where solvent A is 0.05% trifluoroacetic acid (TFA) in water and solvent B is 0.05% TFA in acetonitrile.

The amino acid composition and mass analysis were consistent with the designed sequence. For amino acid analysis, purified peptides were hydrolyzed in 6 N HCl containing 0.1% phenol at 100°C for 24 hours or 1 hour at 160°C in evacuated sealed tubes. Amino acid analysis was performed on a Beckman model 6300 amino acid analyzer (Beckman, San Ramon, CA). The correct primary ion molecular weights of the reduced peptides were confirmed by plasma desorption time of flight mass spectroscopy on a BIOION-20 Nordic (Uppsala, Sweden).

Example 2

Circular Dichroism Measurements

Circular dichroism (CD) spectra were recorded at 20°C on a Jasco J-500C spectropolarimeter (Jasco, Easton, MD) equipped with a Jasco DP-500N data processor and a Lauda (model RMS) water bath (Brinkmann Instruments, Rexdale, Ontario, Canada) for control of the temperature of the cuvette. Constant $N_2$ flushing was employed. The instrument was routinely calibrated with an aqueous solution of recrystallized d-10-(+)-camphorsulfonic acid at 290 nm.

Molar ellipticity at 200 nm is reported as mean residue molar ellipticity ($[\theta]_{220}$, deg • cm$^2$ • dmol$^{-1}$) and calculated from the equation:

$$[\theta] = [\theta]_{obs} \times mrw/10 \times l \times c$$

$[\theta]_{obs}$ is the ellipticity measured in degrees, *mrw* is the mean residue molecular weight (molecular weight of the peptide divided by the number of amino acid residues), *c* is the peptide concentration in grams per milliliter, and *l* is the optical path length of the cell in centimeters. CD spectra were the average of four scans obtained by collecting data at 0.1-nm intervals from 250 to 190 nm.

Peptide concentrations were determined by amino acid analysis. The pH was measured at room temperature.

Example 3

Heterodimer *vs*. Homodimer Formation

Two peptides, EE (SEQ ID NO:1) and KK (SEQ ID NO:2), were synthesized as described in Examples 1 and 4. CD spectra of peptide mixtures of different ratios of the first subunit peptide (EE; SEQ ID NO:1) and the second subunit peptide (KK; SEQ ID NO:2) were measured as described in Example 2, to determine the degree of heterodimer *vs*. homodimer formation.

The peptides were suspended in a solution containing 0.1 M KCl and 50 mM potassium phosphate buffer, pH 7 at 20 °C (reaction buffer). The total peptide concentration (sum of EE and KK concentrations) was 196 $\mu$M for all measurements.

The data show that as the ratio of the peptides is changed from 0:100 to 50:50, the conformation of the peptide mixture is changed from a random coil structure to an $\alpha$-helical structure. An equimolar mixture of the EE and KK peptides displays the double minima at 220 and 208 nm with -31,000 deg $\cdot$ cm$^2$ $\cdot$ dmol$^{-1}$ of mean residue ellipticity at 220 nm, which corresponds to ~100% $\alpha$-helical structure (Hodges, *et al*., 1990), suggesting that the interhelical ionic repulsions which destabilize the homo-stranded coiled-coil provide a driving force for the formation of the hetero-stranded coiled-coil.

These results indicate that the mixture of peptides EE and KK forms a hetero-stranded coiled-coil.

Example 4

Creation of Lactam Bridges

The N- and C-terminal heptads (terminal repeats) were synthesized semi-automatically using a Labortec peptide synthesizer (Bubendorf, Switzerland). Double couplings with 5 equivalents of 2-(1H-benzotriazol-yl)-1,1,3,3-tetramethyluronium hexafluorophosphate (HBTU), 1-hydroxybenzotriazole (HOBt) and Boc amino acids and 7.5 equivalents of N-methylmorpholine (NMM) in N-methylpyrrolidone (NMP) were utilized for each cycle. During each cycle, the Boc group was removed with 50% trifluoroacetic acid (TFA) in methylene chloride (DCM).

Cyclizations involving the side chains of lysine and glutamic acid residues at the N- and C-termini of the coiled coil forming peptides were carried out on the resin using a modified protocol of Felix and co-workers (Felix, *et al*., 1988). In order to facilitate the intramolecular cyclization reaction and avoid the undesired intermolecular reaction, a low substitution level (0.13 mmol per gram of resin) was employed. The $\epsilon$-amino group of Lysines 35 and 7 and the $\gamma$-carboxyl group of glutamic acids 31 and 3 for both peptides were protected with Fmoc and OFm groups, respectively. This allowed for the selective deprotection of these residues with 20% piperidine prior to the solid phase cyclization with 3 equivalents of HBTU, HOBt and 4.5 equivalents of NMM in NMP. The synthesis of the C-terminal heptad of peptide EE, shown in Figure 2, serves to outline the cyclization procedure.

The intervening heptads 2-4 were prepared on a Applied Biosystems 430A peptide synthesizer. All amino acids were double coupled using Dicyclohexylcarbodiimide (DCC) generated symmetric anhydrides (5 equivalents) in dimethyl formamide (DMF) for the first coupling step and DCM for the second coupling step.

A. Preparation of BocLys(Fmoc)-Benzhydrylamine Resin (Labortec SP 640 Peptide Synthesizer)

Benzhydrylamine resin (3.0 g, 0.74 meq/g resin, 2.2 meq) was washed with 30 mL each of DCM, methanol (MeOH), DCM, 5% diisopropylethylamine (DIEA) in DCM ($\times$ 2) DCM, and NMP ($\times$ 2). BocLys(Fmoc) (1.14 g, 2.4 mmol), HBTU (0.91 g, 2.4 mmol), HOBt (0.37 g, 2.4 mmol) were dissolved in NMP (15 mL) to which was added NMM (0.51 mL, 3.63 mmol) and solution was preactivated for 5 minutes. This solution was added to the swelled resin and allowed to sir for 5 minutes. The resultant BocLys(Fmoc) -resin was washed with NMP (2 $\times$ 1 min) and DCM (3 $\times$ 1 min).

B. Preparation of the C- and N-Terminal Heptads

After deprotection (50% TFA in DCM, 1 $\times$ 20 min) and neutralization (5% DIEA in DCM, 2 $\times$ 2 min) the resin was washed with DCM (2 $\times$ 1 min) and NMP (3 $\times$ 1 min). The next amino acid and all following amino acids for the C-terminal heptad and subsequent amino acids of the N-terminal heptad were double coupled according to the following protocol.

Boc amino acid (5 eq.), HBTU (5 eq.), HOBt (5 eq.) were dissolved in NMP (15 mL) to which was added NMM (7.5 eq.) and the solution was allowed to preactivate for 5 minutes. This solution was added to the reaction vessel and allowed to gently agitate for 30 minutes. One cycle of the synthesis consisted of the following operations (10 mL of solvent per gram of resin): 1) 50% TFA in DCM (1 $\times$ 1 min); 2) 50% TFA in DCM (1 $\times$ 20 min); 3) DCM (3 $\times$ 1 min); 4)

5% DIEA in DCM (2 × 2 min); 5) DCM (1 × 1 min); 6) NMP (3 × 1 min); 7) couple (30 min); 8) NMP (3 × 1 min); 9) couple (30 min); 10) NMP (2 × 1 min); 11) DCM (3 × 1 min).

C. Lysine-Glutamic Acid Side Chain Cyclizations

After addition of Boc-Ile, selective deprotection of the Fmoc group of lysine and OFm group of glutamic acid was performed with 20% piperidine in DCM (1 × 20 min) and the resin was subsequently washed with DCM (2 × 1 min) and NMP (3 × 1 min). Cyclizations were performed using the following protocol.

HBTU (3 eq.) HOBT (3 eq.) and NMM (4.5 eq.) were dissolved in NMP to which was added 0.5 mL of hexafluoroisopropanol. The solution was added to the reaction vessel and allowed to gently agitate for 8 hours. The progress of the reaction was monitored by quantitative ninhydrin test (Sarin, *et al*., 1981). Typically, three coupling were required to achieve coupling efficiency of greater than 97%. The resin was acetylated for 1 hour with 10 equivalents of acetic anhydride in 25 mL of 5% DIEA in DCM and washed with DCM, MeOH, DCM and NMP (× 2). The following steps were employed for each cyclization: 1) 20% piperidine in DCM (1 × 1 min); 2) 20% piperidine in DCM (1 × 20 min); 3) DCM (2 × 1 min); 4) NMP (3 × 1 min); 5) couple (8 h); 6) NMP (2 × 1 min); 7) DCM (1 × 1 min); 8) 5% DIEA in DCM (1 × 1 min); 9) DCM (1 × 1 min); NMP (2 × 1 min) 11) couple (3 h); 12) repeat steps 6-10; 13) couple (1 h).

Example 5

Linking Peptide Antigens to Heterodimer Scaffold by Alkylation of Thiol Groups

This example describes conjugation of the Nα-terminal iodacetylated PAK strain pilin antigen (SEQ ID NO:18) to the KK carrier sequence (SEQ ID NO:2).

Prior to conjugation, the Nα-terminus of PAK antigen (SEQ ID NO:18) was extended by the addition of norleucine, an internal marker, and two glycine residues acting as spacers, forming IAc-GG-Nle-PAK. Similarly, the Nα-terminus of the TT2 peptide (SEQ ID NO:12) was extended by the addition of three glycines and a bromoacetyl group, forming BrAc-GGG-TT2. These extensions serve to separate the antigens from the carrier polypeptides, tending to preserve their antigenicity. Extensions like those described above are generally recommended in the synthesis of immunogenic complexes of the present invention.

Conjugation to carrier peptide sulphydryl groups was carried out at ambient temperature in 50 mM $NH_4OAc$ and 8 M urea at pH 8. Bromo- or iodacetylated peptides were dissolved in buffer (0.987 µM, 2 ml) and carrier peptide KK (SEQ ID NO:2) was added to a final concentration of 0.165 µM (2 ml). The reaction mixture remained clear and was allowed to react at ambient temperature for 22 hours, at which time it was acidified by the careful addition of TFA (pH 2) and lyophilized.

A. Conjugate Purification and Identification

The reaction mixture (2 ml) was applied directly to a Synchropak RP-8 semi-prep column (250 mm × 10 mm I.D.; Synchrom Inc., Lafayette, IN). The conjugate was easily separated from unreacted peptide using gradient elution (2% B/minute over 30 minutes, Solvent A: 0.05% $TFA/H_2O$; Solvent B: 0.05% TFA/acetonitrile). The isolated conjugate was lyophilized and redissolved in HPLC grade water (200 µl) which was then applied to a Mono-S strong cation exchange column (Pharmacia, Uppsala, Sweden) for further purification. The gradient employed during this purification step was a 1% B/minute gradient (Solvent A: 5 mM $NaH_2PO_4$/20% acetonitrile, pH 5, Solvent B: 5 mM $NaH_2PO_4$/20% acetonitrile, 1 M NaCl, pH 5). The isolated conjugate was then desalted using a reversed-phase column and a standard 2% B gradient *(vide supra)*. In this way, pure conjugate was obtained which was shown through mass spectrometric analysis to be the desired product (MW calc: 7432.0, Found, 7432.4).

Example 6

Generating Heterodimers by Mixing Conjugated Core-Antigen Monomers

PAK (SEQ ID NO:18) and TT2 (SEQ ID NO:12) peptides were prepared and purified as described in Example 1. EE (SEQ ID NO:1) and KK (SEQ ID NO:2) peptides were prepared, purified and modified as described in Examples 1 and 4. The [PAK]-KK and EE-[TT2] peptide-carrier complexes were prepared as described in Example 5 and purified as detailed in Example 1. Heterodimer complexes were generated by combining the [PAK]-KK complex with the EE-[TT2] complex under the following conditions:

Purified, lyophilized, decorated [PAR]-KK and EE-[TT2] peptides were individually resuspended in reaction buffer, at a concentration of 0.25 - 0.5 mM. 50 µl of each peptide solution were combined and allowed to react for 10 minutes at room temperature.

Example 7

Preparation of Mouse Antibodies Using the [PAK]-KK~EE-[TT2] Heterodimer Complex

Balb C mice (10 animals) are immunized intraperitoneally with a [PAK]-KK-EE-[TT2] heterodimer conjugate mixture comprising 5 µg of the conjugate dissolved in 100 µl of a 1:1 mixture of Adjuvax ADJ-20 (Alfa-Beta Technology, Worcester, MA) and 10 mM phosphate buffered saline (PBS). The injections are given intraperitoneally at one abdominal site. The mice are boosted after 7, 14, and 21 days with the same amount of conjugate in Adjuvax ADJ-20.

Control experiments are performed on 10 animals immunized with 5 µg of conjugate [PAK]-KK prepared as above. Immunizations are conducted in an identical fashion to those described for the test group above.

Sera are tested for immunoreactivity using a standard ELISA protocol, as described in the Materials and Methods. Titers are estimated from reactivity plate ELISA assays using either (i) purified *Pseudomonas aeruginosa* strain K pili or (ii) N-linked synthetic PAK peptide (SEQ ID NO:18) coupled to bovine serum albumin as the solid phase reactive species.

Spleens from three animals are pooled, and processed to produce cells for fusion with myeloma cells, as described in Harlow, *et al.* Hybridoma supernatants are tested for presence of immunoreactive antibodies by ELISA tests with the antigen. Supernatants from positive clones are tested for immunoreactivity with purified *Pseudomonas* derived antigens.

Example 8

Mouse Vaccination Using the [PAK]-KK-EE-[TT2] Heterodimer Complex and Subsequent Protection from Infection by *Pseudomonas aeruginosa*

A. Study 1

Groups of Balb/c mice (5 to 10 animals per group) are immunized with either (i) a control formulation ([PAK]-KK) or (ii) an antigenic coiled-coiled heterodimer formulation ([PAK]-KK~EE-[TT2]). Injections of 1, 5 or 10 µg peptide mixed with adjuvax in phosphate buffered saline are administered to test animals intra-peritoneally (IP) at 0, 2, 4, and 6 weeks. Animals are exsanguinated weekly and the serum is tested for antibody responses. Antibody levels are assessed by direct ELISAs employing (i) purified *Pseudomonas aeruginosa* strain K pili and (ii) N-linked synthetic peptide coupled to bovine serum albumin.

Following the procedures outlined above, titres of $<10^2$ are possible for animals immunized with the control peptide and tested with a peptide-BSA antigen. Even lower titers are possible for control animals tested with a PAK pilin antigen ELISA.

In contrast, immunization with the coiled-coil heterodimer formulation may result in high titres of antibody against both the peptide-BSA conjugate and purified PAX pili. Titers as high as $10^6$ to $10^8$ (for both the peptide and native antigen) are possible after 3 to 4 injections of a 5 µg/injection dose.

B. Study 2

Groups of 5 to 10 AB.Y/SnJ mice (~4 weeks of age) are immunized with adjuvax in buffer, the control peptide and the coiled-coiled formulation in 3 biweekly injections (containing 5 µg of peptide and adjuvax as an adjuvant) intramuscularly (IM). Two weeks after the last immunization, the mice (~ 12 weeks of age) are challenged IP with viable *Pseudomonas aeruginosa* strain K at a dose of $2 \times 10^6$ CFU (a challenge dose equal to $5 \times LD_{50}$). The mice are monitored over the next 60 hours to determine the level of protection afforded by the vaccine formulations against *Pseudomonas aeruginosa* infections.

Control animals (mice immunized with adjuvax or with the adjuvax and the control peptide formulation) may succumb to the *Pseudomonas aeruginosa* infection within 16 to 20 hours experiencing 100% mortality. Mice immunized with the coiled-coiled peptide vaccine formulation may survive the *Pseudomonas aeruginosa* challenge and experience less than 40% mortality.

While the invention has been described with reference to specific methods and embodiments, it will be appreciated that various modifications and changes may be made without departing from the invention.

## Claims

1. A heterodimer polypeptide immunogen, comprising

a carrier having first and second subunits that interact to form a coiled-coil heterodimer, wherein (i) each subunit is derivatized to contain an antigen, (ii) one subunit contains a T-cell antigen and the other subunit contains a B-cell antigen, and (iii) the antigens do not substantially interfere with the formation of the coiled-coil heterodimer.

2. The polypeptide immunogen of claim 1, wherein said coiled-coil heterodimer is stabilized by ionic interactions.

3. The polypeptide immunogen of claim 1 or 2, wherein at least one subunit and its antigen are a single polypeptide chain.

4. The polypeptide immunogen of claim 3, wherein the single polypeptide chain has an amino acid sequence that includes a sequence present in SEQ ID NO:28.

5. The polypeptide immunogen of claim 3, wherein the single polypeptide chain has an amino acid sequence that includes a sequence present in SEQ ID NO:30.

6. The polypeptide immunogen of any of claims 1-5, wherein said T-cell antigen is comprised of a peptide having a sequence selected from the group consisting of SEQ ID NO:10, SEQ ID NO:11, SEQ ID NO:12, SEQ ID NO:13 and SEQ ID NO:14.

7. The polypeptide immunogen of claim 6, wherein said T cell antigen has the sequence represented as SEQ ID NO:12 and said B cell antigen has the sequence represented as SEQ ID NO:18.

8. The polypeptide immunogen of claim 1 or 2, wherein the first subunit comprises

   (A) a first core polypeptide containing (i) two terminal amino acid repeat sequences having the form gabcdef, and (ii) at least one internal amino acid repeat sequence having the form gabcdef, wherein positions a and d of each terminal and internal amino acid repeat sequence are selected from the group consisting of isoleucine, leucine and valine, and positions e and g of each terminal and internal amino acid repeat sequence are selected from the group consisting of aspartic acid and glutamic acid, and
   (B) a first peptide antigen attached to a first core polypeptide internal amino acid repeat sequence through a covalent linkage to an amino acid at position b, c or f; and

   the second subunit comprises

   (A) a second core polypeptide containing (i) two terminal amino acid repeat sequences having the form g'a'b'c'd'e'f', and (ii) at least one internal amino acid repeat sequence having the form g'a'b'c'd'e'f', wherein positions a' and d' of each terminal and internal amino acid repeat sequence are selected from the group consisting of isoleucine, leucine and valine, positions e' and g' of each terminal and internal amino acid repeat sequence are selected from the group consisting of lysine, arginine and histidine, and
   (B) a second peptide antigen attached to a second core polypeptide internal amino acid repeat sequence through a covalent linkage to an amino acid at position b', c' or f';
   wherein said first and second subunits are arranged in a stable $\alpha$-helical coiled-coil configuration having a 1:1 stoichiometry, and wherein the peptide antigens are disposed toward outer surfaces of the configuration.

9. The polypeptide immunogen of claim 8, wherein

   (i) the terminal amino acid repeat sequences of the first core polypeptide each have a glutamic acid at position b, a lysine at position f and a lactam bridge formed between said positions b and f, and
   (ii) the terminal amino acid repeat sequences of the second core polypeptide each have a glutamic acid at position b,' a lysine at position f' and a lactam bridge formed between said positions b' and f'.

10. The polypeptide immunogen of claim 8, wherein

    the first core polypeptide includes an amino acid coupling residue at position f in at least one of its internal repeat sequences, and
    the second core polypeptide includes an amino acid coupling residue at position f' in at least one of its internal amino acid repeat sequences.

**11.** The polypeptide immunogen of claim 10, wherein said amino acid coupling residue is a cysteine residue.

**12.** The polypeptide immunogen of claim 11, wherein the first core polypeptide has the sequence SEQ ID NO:1 (EE), and the second core polypeptide has the sequence SEQ ID NO:2 (KK).

**13.** A method of preparing a heterodimer polypeptide immunogen composition of claim 1, comprising

forming two polypeptide subunits that interact to form a coiled-coil heterodimer, wherein (i) each subunit is derivatized to contain an antigen, (ii) one subunit contains a T-cell antigen and the other subunit contains a B-cell antigen, and (iii) the antigens do not substantially interfere with the formation of the coiled-coil heterodimer, and mixing said polypeptide subunits in a benign medium in a ratio of about 1:1 under conditions that promote formation of said coiled-coil heterodimer.

**14.** The method of claim 13, wherein said coiled-coil heterodimer is stabilized by ionic interactions.

**15.** The method of claim 13 or 14, wherein said T-cell antigen is comprised of a peptide selected from the group consisting of SEQ ID NO:10, SEQ ID NO:11, SEQ ID NO:12, SEQ ID NO:13 and SEQ ID NO:14.

**Patentansprüche**

**1.** Heterodimeres Polypeptidimmunogen, umfassend einen Träger, der erste und zweite Untereinheiten hat, welche unter Bildung eines Heterodimers als gewundenes Knäuel ("coiled-coil") interagieren wobei (i) jede Untereinheit so derivatisiert ist, daß sie ein Antigen enthält, (ii) eine Untereinheit ein T-Zell-Antigen und die andere Untereinheit ein B-Zell-Antigen enthält und (iii) die Antigene die Bildung des Heterodimers als gewundenes Knäuel nicht wesentlich stören.

**2.** Polypeptidimmunogen nach Anspruch 1, wobei das Heterodimer als gewundenes Knäuel durch ionische Wechselwirkungen stabilisiert ist.

**3.** Polypeptidimmunogen nach Anspruch 1 oder 2, wobei mindestens eine Untereinheit und ihr Antigen eine einzelne Polypeptidkette sind.

**4.** Polypeptidimmunogen nach Anspruch 3, wobei die einzelne Polypeptidkette eine Aminosäuresequenz hat die eine Sequenz beinhaltet wie sie in SEQ ID Nr. 28 vorliegt.

**5.** Polypeptidimmunogen nach Anspruch 3, wobei die einzelne Polypeptidkette eine Aminosäuresequenz hat, die eine Sequenz beinhaltet, wie sie in SEQ ID Nr. 30 vorliegt.

**6.** Polypeptidimmunogen nach einem der Ansprüche 1 bis 5, wobei das T-Zell-Antigen ein Peptid umfaßt, das eine Sequenz hat, ausgewählt aus der Gruppe SEQ ID Nr. 10, SEQ ID Nr. 11, SEQ ID Nr. 12, SEQ ID Nr. 13 und SEQ ID Nr. 14.

**7.** Polypeptidimmunogen nach Anspruch 6, wobei das T-Zell-Antigen die als SEQ ID Nr. 12 dargestellte Sequenz hat und das B-Zell-Antigen die als SEQ ID Nr. 18 dargestellte Sequenz hat.

**8.** Polypeptidimmunogen nach Anspruch 1 oder 2, die erste Untereinheit umfassend

(A) ein erstes Kernpolypeptid, beinhaltend (i) zwei terminale Aminosäurewiederholungssequenzen, welche die Form gabcdef haben, und (ii) mindestens eine innere Aminosäurewiederholungssequenz, welche die Form gabcdef hat, wobei die Position a und d jeder terminalen und inneren Aminosäurewiederholungssequenz aus der Gruppe bestehend aus Isoleucin, Leucin und Valin ausgewählt sind und die Positionen e und g jeder terminalen und inneren Aminosäurewiederholungssequenz aus der Gruppe bestehend aus Asparaginsäure und Glutaminsäure ausgewählt sind, und
(B) ein erstes Peptidantigen, über eine kovalente Bindung an eine Aminosäure an Position b, c oder f einer inneren Aminosäurewiederholungssequenz eines Kernpolypeptids befestigt, und

die zweite Untereinheit umfassend

(A) ein zweites Kernpolypeptid beinhaltend (i) zwei terminale Aminosäurewiederholungssequenzen, welche die Form g'a'b'c'd'e'f' haben, und (ii) mindestens eine innere Aminosäurewiederholungssequenz, welche die Form g'a'b'c'd'e'f' hat, wobei die Positionen a' und d' jeder terminalen und inneren Aminosäurewiederholungssequenz ausgewählt sind aus der Gruppe bestehend aus Isoleucin, Leucin und Valin, Positionen e' und g' jeder terminalen und inneren Aminosäurewiederholungssequenz ausgewählt sind aus der Gruppe bestehend aus Lysin, Arginin und Histidin und

(B) ein zweites Peptidantigen, über eine kovalente Bindung an eine Aminosäure an Position b', c' oder f' einer inneren Aminosäurewiederholungssequenz eines zweiten Kempolypeptids befestigt;

wobei die ersten und zweiten Untereinheiten in einer stabilen α-helicalen Konfiguration als gewundenes Knäuel angeordnet sind, die eine 1:1-Stöchiometrie hat und wobei die Peptidantigene zu äußeren Flächen der Konfiguration hin angeordnet sind.

9. Polypeptidimmunogen nach Anspruch 8, wobei

(i) jede der terminalen Aminosäurewiederholungssequenzen des ersten Kernpolypeptids eine Glutaminsäure an Position b, ein Lysin an Position f und eine zwischen den Positionen b und f ausgebildete Lactam-Brücke hat, und

(ii) jede der terminalen Aminosäurewiederholungssequenzen des zweiten Kernpolypeptids eine Glutaminsäure an Position b', ein Lysin an Position f' und eine zwischen den Positionen b' und f' ausgebildete Lactam- Brücke hat.

10. Polypeptidimmunogen nach Anspruch 8, wobei das erste Kernpolypeptid in mindestens einer seiner inneren Wiederholungssequenzen an Position f einen Aminosäurekupplungsrest enthält und das zweite Kernpolypeptid in mindestens einer seiner inneren Aminosäurewiederholungssequenzen an Position f' einen Aminosäurekupplungsrest enthält.

11. Polypeptidimmunogen nach Anspruch 10, wobei der Aminosäurekupplungsrest ein Cysteinrest ist.

12. Polypeptidimmunogen nach Anspruch 11, wobei das erste Kernpolypeptid die Sequenz SEQ ID Nr. 1 (EE) und das zweite Kernpolypeptid die Sequenz SEQ ID Nr. 2 (KK) hat.

13. Verfahren zur Herstellung einer heterodimeren Polypeptidimmunogen-Zusammensetzung nach Anspruch 1, umfassend

Ausbilden zweier Polypeptiduntereinheiten, welche unter Bildung eines Heterodimers als gewundenes Knäuel interagieren wobei (i) jede Untereinheit so derivatisiert ist, daß sie ein Antigen enthält, (ii) eine Untereinheit ein T- Zell-Antigen enthält und die andere Untereinheit ein B-Zell-Antigen enthält und (iii) die Antigene die Ausbildung des Heterodimers als gewundenes Knäuel nicht wesentlich stören, und

Mischen der Polypeptiduntereinheiten in einem geeignetem Medium im Verhältnis 1:1 unter Bedingungen, welche die Ausbildung des Heterodimers als gewundenes Knäuel fördern.

14. Verfahren nach Anspruch 13, wobei das Heterodimer als gewundenes Knäuel durch ionische Wechselwirkungen stabilisiert ist.

15. Verfahren nach Anspruch 13 oder 14, wobei das T-Zell-Antigen ein Peptid umfaßt, ausgewählt aus der Gruppe bestehend aus SEQ ID Nr. 10, SEQ ID Nr. 11, SEQ ID Nr. 12, SEQ ID Nr. 13 und SEQ ID Nr. 14.

**Revendications**

1. Immunogène polypeptidique hétérodimérique, comprenant un support ayant une première et une seconde sous- unités qui interagissent pour former un hétérodimère superenroulé, dans lequel (i) chaque sous-unité est dérivée de manière à contenir un antigène, (ii) une sous-unité contient un antigène des oellules T et l'autre sous-unité contient un antigène des cellules B, et (iii) les antigènes n'interfèrent pratiquement pas avec la formation de l'hétérodimère superenroulé.

2. Immunogène polypeptidique selon la revendication 1, dans lequel ledit hétérodimère superenroulé est stabilisé par des interactions ioniques.

3. Immunogène polypeptidique selon l'une des revendications 1 ou 2, dans lequel au moins une sous-unité et son antigène sont une chaîne simple polypeptidique.

4. Immunogène polypeptidique selon la revendication 3, dans lequel la chaîne simple polypeptidique a une séquence d'acides aminés qui inclut une séquence présente dans SEC ID n⁰ 28.

5. Immunogène polypeptidique selon la revendication 3, dans lequel la chaîne simple polypeptidique a une séquence d'acides aminés qui inclut une séquence présente dans SEQ ID n⁰ 30.

6. Immunogène polypeptidique selon l'une quelconque des revendications 1 à 5, dans lequel ledit antigène des cellules T comprend un peptide ayant une séquence choisie parmi SEC ID n⁰ 10, SEQ ID n⁰ 11, SEQ ID n⁰ 12, SEQ ID n⁰ 13 et SEQ ID n⁰ 14.

7. Immunogène polypeptidique selon la revendication 6, dans lequel ledit antigène des cellules T a la séquence représentée SEQ ID n⁰ 12 et ledit antigène des cellules B a la séquence représentée SEQ ID n⁰ 18.

8. Immunogène polypeptidique selon l'une des revendications 1 ou 2, dans lequel la première sous-unité comprend:

(A) un premier polypeptide central contenant (i) deux séquences de répétition d'acides aminés terminales ayant la forme gabcdef et (ii) au moins une séquence de répétition d'acides aminés interne ayant la forme gabcdef, les positions a et d de chaque séquence de répétition d'acides aminés interne et terminale étant choisies parmi l'isoleucine, la leucine et la valine, et les positions e et g de chaque séquence de répétition d'acides aminés interne et terminale étant choisies parmi l'acide aspartique et l'acide glutamique, et

(B) un premier antigène peptidique lié à une séquence de répétition d'acides aminés interne du premier polypeptide central par une liaison covalente d'un acide aminé en positions b, c ou f; et

la seconde sous-unité comprend:

(A) un second polypeptide central contenant (i) deux séquences de répétition d'acides aminés terminales ayant la forme g'a'b'c'd'e'f', et (ii) au moins une séquence de répétition d'acides aminés interne ayant la forme g'a'b'c'd'e'f', les positions a' et d' de chaque séquence de répétition d'acides aminés terminale et interne étant choisies parmi l'isoleucine, la leucine et la valine, les positions e' et g' de chaque séquence de répétition d'acides aminés terminale et interne étant choisies parmi la lysine, l'arginine et l'histidine, et

(B) un second antigène peptidique lié à une séquence de répétition d'acides aminés interne du second polypeptide central par une liaison covalente d'un acide aminé en position b', c' ou f';

lesdites première et seconde sous-unités étant présentées dans une configuration stable d'hélice-α superenroulée ayant une stoechiométrie 1:1, et les antigènes peptidiques étant disposés vers les surfaces externes de la configuration.

9. Immunogène polypeptidique selon la revendication 8, dans lequel

(i) les séquences de répétition d'acides aminés terminales du premier polypeptide central ont chacune un acide glutamique en position b, une lysine en position f et un pont lactame formé entre lesdites positions b et f, et

(ii) les séquences de répétition d'acides aminés terminales du second polypeptide central ont chacune un acide glutamique en position b', une lysine en position f' et un pont lactame formé entre lesdites positions b' et f'.

10. Immunogène polypeptidique selon la revendicetion 8, dans lequel

le premier polypeptide central inclut un résidu de couplage d'acides aminés en position f dans au moins une de ses séquences de répétition interne, et

le second polypeptide central inclut un résidu de couplage d'acides aminés en position f' dans au moins une de ses séquences de répétition d'acides aminés interne.

11. Immunogène polypeptidique selon la revendication 10, dans lequel ledit résidu de couplage d'acides aminés est un résidu de cystéine.

12. Immunogène polypeptidique selon la revendication 11, dans lequel le premier polypeptide central a la séquence

SEQ ID n° 1 (EE), et le second polypeptide central a la séquence SEQ ID n° 2 (KK).

**13.** Méthode de préparation d'une composition à base d'immunogène polypeptidique hétérodimérique de la revendication 1 comprenant

la formation de deux sous-unités polypeptidiques qui interagissent pour former un hétérodimère superenroulé, dans laquelle (i) chaque sous-unité est dérivée de manière à contenir un antigène, (ii) une sous-unité contient un antigène des cellules T et l'autre sous-unité contient un antigène des cellules B, et (iii) les antigènes n'interfèrent pratiquement pas avec la formation de l'hétérodimère superenroulé, et

le mélange desdites sous-unités polypeptidiques dans un milieu minimal dans un rapport d'environ 1:1 dans des conditions qui permettent la formation dudit hétérodimère superenroulé.

**14.** Méthode selon la revendication 13, dans laquelle ledit hétérodimère superenroulé est stabilisé par des interactions ioniques.

**15.** Méthode selon l'une des revendications 13 ou 14, dans laquelle ledit antigène des cellules T comprend un peptide choisi parmi SEQ ID n° 10, SEQ ID n° 11, SEQ ID n° 12, SEQ ID n° 13 et SEQ ID n° 14.

## Fig. 1A

CP1

CP2

## Fig. 1B

Ag1

Ag1-CP1

Ag2

CP2-Ag2

Ag1

Ag2

**Fig. 1C**

Ag1-CP1~CP2-Ag2

**PARALLEL**

Fig. 2A

Fig. 2B

# Homodimers          # Heterodimers

**Stabilized**

## Fig. 3A

**Destabilized**

## Fig. 3B

**Destabilized**

## Fig. 3C

**Stabilized**

## Fig. 3D

**Destabilized**

## Fig. 3E

CP1 CP2

**Fig. 4A**

CP1 CP2

**Fig. 4B**

CP1 CP2

**Fig. 4C**

**Fig. 5**

TTAGGCACCCCAGGCTTTACACTTTATGCTTCCGGCTCGTATGTTGTGTGGAATTGTGAGCGG

                                                   **Xba I**
ATAACAATTTCACACAGGAAACAGCT ATG ACC ATG ATT ACG AAT TTC TAG ATAACGAG
                              M   T   M   I   T   N  F  End
                              lac I -->
GGCAAAAA ATG AAA AAG ACA GCT ATC GCG ATT GCA GTG GCA CTG GCT GGT TTC GCT
          M   K   K   T   A   I   A   I   A   V   A   L   A   G   F   A
      ompA -->
                      **Xho I**    **Kpn I**    **EcoRI**
ACC GTA GCG CAG GCC GCG CTC GAG GGT ACC GAA TTC GGA GGA GGT GGA GGT GGT
 T   V   A   Q   A   A   L   E   G   T   E   F   G   G   G   G   G   G
                                             **Coiled and coil -->**
GGT GGC GAG GTA TCC GCT TTA GAG AAA GAA GTT TCT GCT CTC GAA AAA GAG GTC
 G   G   E   V   S   A   L   E   K   E   V   S   A   L   E   K   E   V

AGT GCT CTG GAA AAA GAG GTG TCA GCC TTG GAA AAG GAA GTA TCA GCA CTT GAG
 S   A   L   E   K   E   V   S   A   L   E   K   E   V   S   A   L   E

AAG GGC GGT GGA GGA CAT CAC CAC CAT CAC TAA TAA GGA TCT GCGGCCGCAC
 K   G   G   G   G   H   H   H   H   H   *    *          **Not I**

AA GCT TGA CCT GTG AAG TGA AAA ATG GCG CAC ATT GTG CGA CAT TTT TTT TGT
**Hind III**

                            **Bam HI**
CTG CCG TTT.ACC GCT ACT GCG TCA CGG ATC C

**Fig. 6**

## Fig. 7

TTAGGCACCCCAGGCTTTACACTTTATGCTTCCGGCTCGTATGTTGTGTGGAATTGTGAGCGG

ATAACAATTTCACACAGGAAACAGCT ATG ACC ATG ATT ACG AAT TTC TAG ATAACGAG
           M T M I T N F End
          lac I -->

GGCAAAAA ATG AAA AAG ACA GCT ATC GCG ATT GCA GTG GCA CTG GCT GGT TTC GCT
     M K K T A I A I A V A L A G F A
  ompA -->

           Xho I  Kpn I  EcoRI
ACC GTA GCG CAG GCC GCG CTC GAG GGT ACC GAA TTC GGA GGA GGT GGA GGT GGT
T V A Q A A L E G T E F G G G G G
                       Coiled and coil -->
GGT GGC AAG GTA TCC GCT TTA AAA GAG AAA GTT TCT GCT CTG AAA GAA AAG GTC
G G K V S A L K E K V S A L K E K V

AGT GCT CTG AAG GAG AAG GTG TCA GCC TTG AAG GAA AAG GTT TCA GCA CTT AAA
S A L K E K V S A L K E K V S A L K

GAG GGC GGT GGA GGA CAT CAC CAC CAT CAC TAA TAA GGA TCT GCGGCCGCAC
E G G G G H H H H H * *     Not I

AA GCT TGA CCT GTG AAG TGA AAA ATG GCG CAC ATT GTG CGA CAT TTT TTT TGT
Hind III

CTG CCG TTT ACC GCT ACT GCG TCA CGG ATC C

## Fig. 8

**Fig. 9**

ACAGGCAATAATATAAACAGAAGGAAGCTGCCCTGTCTTAAACCTTTTTTTTTTATCATCATTATT

GCTTACTTTCATAATTGCGACTGGTTCCAATTGACAAGCTTTTGATTTTAACGACTTTTAACGAC

AAACTTGAGAAGATCAAAAAAACAACTAATTATTCGAAACG ATG TTC TCT CCA ATT TTC TCC

                                            **Xho I   EcoRI**

TTG GAA ATT ATT TTA GCT TTG GCT ACT TTGCCA TCT GTC TTC GCT CGA GAA TTC

                                              **E     F**

                                  **SIGNAL   CLEAVAGE**

GGA GGA GGT GGA GGT GGT GGT GGC GAG GTA TCC GCT TTA GAG AAA GAA GTT TCT
  G    G    G    G    G    G    G    G    E    V    S    A    L    E    K    E    V    S
Coiled and coil -->

GCT CTC GAA AAA GAG GTC AGT GCT CTG GAA AAA GAG GTG TCA GCC TTG GAA AAG
A    L    E    K    E    V    S    A    L    E    K    E    V    S    A    L    E    K

GAA GTA TCA GCA CTT GAG AAG GGC GGT GGA GGA CAT CAC CAC CAT CACTAA TAA
E    V    S    A    L    E    K    G    G    G    H    H    H    H    H    *    *

GGA TCC
BamHI

**Fig. 10**

Fig. 11

ACAGGCAATATATAAACAGAAGGAAGCTGCCCTGTCTTAAACCTTTTTTTTTATCATCATTATTAGCTTACTTTCA
TAATTGCGACTGGTTCCAATTGACAAGCTTTTGATTTTAACGACAACTTGAGAAGATCAAAAAACAACTAATTATT

PHO1 Start
CGAAACG ATG TTC TCT CCA ATT TTG TCC TTG GAA ATT ATT TTA GCT TTG GCT ACT TTG CAA TCT GTC
    M  F  S  P  I  L  S  L  E  I  I  L  A  L  A  T  L  Q  S  V

Xho I
TTC GCT CGA GAA TTC GGA GGA GGT GGA GGT GGT GGT GGC AAG GTA TCC GCT TTA AAA
F  A  ^R  E  F  G  G  G  G  G  G  G  K  V  S  A  L  K
Signal cleavage  Coiled and coil -->

GAGAAA GTT TCT GCT CTG AAA GAA AAG GTC AGT GCT CTG AAG GAG AAG GTG TCA
E  K  V  S  A  L  K  E  K  V  S  A  L  K  L  K  V  S

GCC TTG AAG GAA AAG GTT TCA GCA CTT AAA GAG GGC GGT GGA GGA CAT CAC CAC
A  L  K  E  K  V  S  A  L  K  E  G  G  G  G  H  H  H

CAT CAC TAA TAA GGA TCC
       BamHI

# Fig. 12

EP 0 759 941 B1

```
1/1                                              31/11
cga gaa ttc aag tgt act tct gac caa gac gag caa ttc atc cct aag ggt tgt tcc aaa
R   E   F   K   C   T   S   D   Q   D   E   Q   F   I   P   K   G   C   S   K
61/21                                            91/31
ttc gga gga ggt gga ggt ggt ggt ggc gag gta tcc gct tta gag aaa gaa gtt tct gct
F   G   G   G   G   G   G   G   E   V   S   A   L   E   K   E   V   S   A
121/41                                           151/51
ctc gaa aaa gag gtc agt gct ctg gaa aaa gag gtg tca gcc ttg gaa aag gaa gta tca
L   E   K   E   V   S   A   L   E   K   E   V   S   A   L   E   K   E   V   S
181/61                                           211/71
gca ctt gag aag ggc ggt gga gga cat cac cac cat cac taa taa gga tcc
A   L   E   K   G   G   G   G   H   H   H   H   H   *   *   G   S
```

# Fig. 13

54

```
1/1                                             31/11
cga gaa ttc ttg tct gag atc aag gga gta atc gtc cac aga ctt gaa ggt gtc aaa ttc
R   E   F   L   S   E   I   K   G   V   I   V   H   R   L   E   G   V   K   F
61/21                                           91/31
gga gga ggt gga ggt ggt ggt ggc gag gta tcc gct tta gag aaa gaa gtt tct gct ctc
G   G   G   G   G   G   G   G   E   V   S   A   L   E   K   E   V   S   A   L
121/41                                          151/51
gaa aaa gag gtc agt gct ctg gaa aaa gag gtg tca gcc ttg gaa aag gaa gta tca gca
E   K   E   V   S   A   L   E   K   E   V   S   A   L   E   K   E   V   S   A
181/61                                          211/71
ctt gag aag ggc ggt gga gga cat cac cac cat cac taa taa gga tcc
L   E   K   G   G   G   G   H   H   H   H   H   *   *   G   S
```

# Fig. 14

EP 0 759 941 B1


SEQUENCE LISTING

(1) GENERAL INFORMATION:

    (i) APPLICANT: S.P.I. Synthetic Peptides Incorporated

    (ii) TITLE OF INVENTION: Heterodimer Polypeptide Immunogen Carrier
        Composition and Method

   (iii) NUMBER OF SEQUENCES: 30

    (iv) CORRESPONDENCE ADDRESS:
        (A) ADDRESSEE: Dehlinger & Associates
        (B) STREET: 350 Cambridge Avenue, Suite 250
        (C) CITY: Palo Alto
        (D) STATE: CA
        (E) COUNTRY: USA
        (F) ZIP: 94306

    (v) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25

    (vi) CURRENT APPLICATION DATA:
        (A) APPLICATION NUMBER:
        (B) FILING DATE: 18-MAY-1995
        (C) CLASSIFICATION:

   (vii) PRIOR APPLICATION DATA:
        (A) APPLICATION NUMBER: US 08/245,507
        (B) FILING DATE: 18-MAY-1994

  (viii) ATTORNEY/AGENT INFORMATION:
        (A) NAME: Sholtz, Charles K.
        (B) REGISTRATION NUMBER: 38,615
        (C) REFERENCE/DOCKET NUMBER: 8900-0009.41

    (ix) TELECOMMUNICATION INFORMATION:
        (A) TELEPHONE: (415) 324-0880
        (B) TELEFAX: (415) 324-0960

(2) INFORMATION FOR SEQ ID NO:1:

    (i) SEQUENCE CHARACTERISTICS:
        (A) LENGTH: 35 amino acids
        (B) TYPE: amino acid
        (C) STRANDEDNESS: single
        (D) TOPOLOGY: unknown

    (ii) MOLECULE TYPE: peptide

    (iii) HYPOTHETICAL: NO

    (iv) ANTI-SENSE: NO

    (vi) ORIGINAL SOURCE:
        (C) INDIVIDUAL ISOLATE: EE peptide

    (xi) SEQUENCE DESCRIPTION: SEQ ID NO:1:

Glu Val Glu Ala Leu Gln Lys Glu Val Ser Ala Leu Glu Lys Glu Val
1             5                 10                15

Ser Ala Leu Glu Cys Glu Val Ser Ala Leu Glu Lys Glu Val Glu Ala
            20                25              30

Leu Gln Lys
    35

(2) INFORMATION FOR SEQ ID NO:2:

    (i) SEQUENCE CHARACTERISTICS:
        (A) LENGTH: 35 amino acids
        (B) TYPE: amino acid
        (C) STRANDEDNESS: single
        (D) TOPOLOGY: unknown

    (ii) MOLECULE TYPE: peptide

    (iii) HYPOTHETICAL: NO

    (iv) ANTI-SENSE: NO

    (vi) ORIGINAL SOURCE:

(C) INDIVIDUAL ISOLATE: KK peptide

(xi) SEQUENCE DESCRIPTION: SEQ ID NO:2:

Lys Val Glu Ala Leu Lys Lys Lys Val Ser Ala Leu Lys Glu Lys Val
1               5               10              15

Ser Ala Leu Lys Cys Lys Val Ser Ala Leu Lys Glu Lys Val Glu Ala
            20              25              30

Leu Lys Lys
        35

(2) INFORMATION FOR SEQ ID NO:3:

    (i) SEQUENCE CHARACTERISTICS:
        (A) LENGTH: 7 amino acids
        (B) TYPE: amino acid
        (C) STRANDEDNESS: single
        (D) TOPOLOGY: unknown

    (ii) MOLECULE TYPE: peptide

    (iii) HYPOTHETICAL: NO

    (iv) ANTI-SENSE: NO

    (vi) ORIGINAL SOURCE:
        (C) INDIVIDUAL ISOLATE: EE terminal repeat

    (xi) SEQUENCE DESCRIPTION: SEQ ID NO:3:

Glu Val Glu Ala Leu Glu Lys
1               5

(2) INFORMATION FOR SEQ ID NO:4:

    (i) SEQUENCE CHARACTERISTICS:
        (A) LENGTH: 7 amino acids
        (B) TYPE: amino acid
        (C) STRANDEDNESS: single
        (D) TOPOLOGY: unknown

          (ii) MOLECULE TYPE: peptide

         (iii) HYPOTHETICAL: NO

          (iv) ANTI-SENSE: NO

          (vi) ORIGINAL SOURCE:
               (C) INDIVIDUAL ISOLATE: EE internal repeat

          (xi) SEQUENCE DESCRIPTION: SEQ ID NO:4:

          Glu Val Ser Ala Leu Glu Lys
          1               5

(2) INFORMATION FOR SEQ ID NO:5:

          (i) SEQUENCE CHARACTERISTICS:
               (A) LENGTH: 7 amino acids
               (B) TYPE: amino acid
               (C) STRANDEDNESS: single
               (D) TOPOLOGY: unknown

          (ii) MOLECULE TYPE: peptide

         (iii) HYPOTHETICAL: NO

          (iv) ANTI-SENSE: NO

          (vi) ORIGINAL SOURCE:
               (C) INDIVIDUAL ISOLATE: EE conjugation internal repeat

          (xi) SEQUENCE DESCRIPTION: SEQ ID NO:5:

          Glu Val Ser Ala Leu Glu Cys
          1               5

(2) INFORMATION FOR SEQ ID NO:6:

          (i) SEQUENCE CHARACTERISTICS:
               (A) LENGTH: 7 amino acids
               (B) TYPE: amino acid
               (C) STRANDEDNESS: single

```
            (D) TOPOLOGY: unknown

       (ii) MOLECULE TYPE: peptide

       (iii) HYPOTHETICAL: NO

       (iv) ANTI-SENSE: NO

       (vi) ORIGINAL SOURCE:
            (C) INDIVIDUAL ISOLATE: KK terminal repeat


       (xi) SEQUENCE DESCRIPTION: SEQ ID NO:6:

       Lys Val Glu Ala Leu Lys Lys
       1               5

(2) INFORMATION FOR SEQ ID NO:7:

       (i) SEQUENCE CHARACTERISTICS:
            (A) LENGTH: 7 amino acids
            (B) TYPE: amino acid
            (C) STRANDEDNESS: single
            (D) TOPOLOGY: unknown

       (ii) MOLECULE TYPE: peptide

       (iii) HYPOTHETICAL: NO

       (iv) ANTI-SENSE: NO

       (vi) ORIGINAL SOURCE:
            (C) INDIVIDUAL ISOLATE: KK internal repeat


       (xi) SEQUENCE DESCRIPTION: SEQ ID NO:7:

       Lys Val Ser Ala Leu Lys Glu
       1               5

(2) INFORMATION FOR SEQ ID NO:8:

       (i) SEQUENCE CHARACTERISTICS:
            (A) LENGTH: 7 amino acids
```

24

(B) TYPE: amino acid

(C) STRANDEDNESS: single

(D) TOPOLOGY: unknown

(ii) MOLECULE TYPE: peptide

(iii) HYPOTHETICAL: NO

(iv) ANTI-SENSE: NO

(vi) ORIGINAL SOURCE:

(C) INDIVIDUAL ISOLATE: KK conjugation internal repeat

(xi) SEQUENCE DESCRIPTION: SEQ ID NO:8:

Lys Val Ser Ala Leu Lys Cys
1               5

(2) INFORMATION FOR SEQ ID NO:9:

(i) SEQUENCE CHARACTERISTICS:

(A) LENGTH: 12 amino acids

(B) TYPE: amino acid

(C) STRANDEDNESS: single

(D) TOPOLOGY: unknown

(ii) MOLECULE TYPE: peptide

(iii) HYPOTHETICAL: NO

(iv) ANTI-SENSE: NO

(vi) ORIGINAL SOURCE:

(C) INDIVIDUAL ISOLATE: B antigen; Exo S peptide

(xi) SEQUENCE DESCRIPTION: SEQ ID NO:9:

Cys Ala Thr Thr Ala Thr Gly Pro Asn Gly Ser Cys
1               5                       10

(2) INFORMATION FOR SEQ ID NO:10:

(i) SEQUENCE CHARACTERISTICS:
    (A) LENGTH: 12 amino acids
    (B) TYPE: amino acid
    (C) STRANDEDNESS: single
    (D) TOPOLOGY: unknown

(ii) MOLECULE TYPE: peptide

(iii) HYPOTHETICAL: NO

(iv) ANTI-SENSE: NO

(vi) ORIGINAL SOURCE:
    (C) INDIVIDUAL ISOLATE: T antigen, TTO peptide

(xi) SEQUENCE DESCRIPTION: SEQ ID NO:10:

Leu Gln Thr Met Val Lys Leu Phe Asn Arg Ile Lys
1               5                   10

(2) INFORMATION FOR SEQ ID NO:11:

(i) SEQUENCE CHARACTERISTICS:
    (A) LENGTH: 20 amino acids
    (B) TYPE: amino acid
    (C) STRANDEDNESS: single
    (D) TOPOLOGY: unknown

(ii) MOLECULE TYPE: peptide

(iii) HYPOTHETICAL: NO

(iv) ANTI-SENSE: NO

(vi) ORIGINAL SOURCE:
    (C) INDIVIDUAL ISOLATE: T antigen, TT peptide

(xi) SEQUENCE DESCRIPTION: SEQ ID NO:11:

Asn Ser Val Asp Asp Ala Leu Ile Asn Ser Thr Lys Ile Tyr Ser Tyr
1               5                   10                  15

26

Phe Pro Ser Val
                 20

(2) INFORMATION FOR SEQ ID NO:12:

    (i) SEQUENCE CHARACTERISTICS:
        (A) LENGTH: 17 amino acids
        (B) TYPE: amino acid
        (C) STRANDEDNESS: single
        (D) TOPOLOGY: unknown

    (ii) MOLECULE TYPE: peptide

    (iii) HYPOTHETICAL: NO

    (iv) ANTI-SENSE: NO

    (vi) ORIGINAL SOURCE:
        (C) INDIVIDUAL ISOLATE: T antigen, TT2 peptide

    (xi) SEQUENCE DESCRIPTION: SEQ ID NO:12:

Gln Tyr Ile Lys Ala Asn Ser Lys Phe Ile Gly Ile Thr Glu Leu Lys
1               5                   10                  15

Lys

(2) INFORMATION FOR SEQ ID NO:13:

    (i) SEQUENCE CHARACTERISTICS:
        (A) LENGTH: 17 amino acids
        (B) TYPE: amino acid
        (C) STRANDEDNESS: single
        (D) TOPOLOGY: unknown

    (ii) MOLECULE TYPE: peptide

    (iii) HYPOTHETICAL: NO

    (iv) ANTI-SENSE: NO

    (vi) ORIGINAL SOURCE:

(C) INDIVIDUAL ISOLATE: T antigen, TT1 peptide

(xi) SEQUENCE DESCRIPTION: SEQ ID NO:13:

Pro Gly Ile Asn Gly Lys Ala Ile His Leu Val Asn Asn Glu Ser Ser
1               5               10              15

Glu

(2) INFORMATION FOR SEQ ID NO:14:

    (i) SEQUENCE CHARACTERISTICS:
        (A) LENGTH: 21 amino acids
        (B) TYPE: amino acid
        (C) STRANDEDNESS: single
        (D) TOPOLOGY: unknown

    (ii) MOLECULE TYPE: peptide

    (iii) HYPOTHETICAL: NO

    (iv) ANTI-SENSE: NO

    (vi) ORIGINAL SOURCE:
        (C) INDIVIDUAL ISOLATE: T antigen, TT3 peptide

    (xi) SEQUENCE DESCRIPTION: SEQ ID NO:14:

Phe Asn Asn Phe Thr Val Ser Phe Trp Leu Arg Val Pro Lys Val Ser
1               5               10              15

Ala Ser His Leu Glu
                20

(2) INFORMATION FOR SEQ ID NO:15:

    (i) SEQUENCE CHARACTERISTICS:
        (A) LENGTH: 15 amino acids
        (B) TYPE: amino acid
        (C) STRANDEDNESS: single
        (D) TOPOLOGY: unknown

(ii) MOLECULE TYPE: peptide

(iii) HYPOTHETICAL: NO

(iv) ANTI-SENSE: NO

(vi) ORIGINAL SOURCE:
    (C) INDIVIDUAL ISOLATE: T antigen, MVF peptide

(xi) SEQUENCE DESCRIPTION: SEQ ID NO:15:

Leu Ser Glu Ile Lys Gly Val Ile Val His Arg Leu Glu Gly Val
1          5               10            15

(2) INFORMATION FOR SEQ ID NO:16:

(i) SEQUENCE CHARACTERISTICS:
    (A) LENGTH: 15 amino acids
    (B) TYPE: amino acid
    (C) STRANDEDNESS: single
    (D) TOPOLOGY: unknown

(ii) MOLECULE TYPE: peptide

(iii) HYPOTHETICAL: NO

(iv) ANTI-SENSE: NO

(vi) ORIGINAL SOURCE:
    (C) INDIVIDUAL ISOLATE: T antigen, HBV peptide

(xi) SEQUENCE DESCRIPTION: SEQ ID NO:16:

Phe Phe Leu Leu Thr Arg Ile Leu Thr Ile Pro Gln Ser Leu Asp
1          5               10            15

(2) INFORMATION FOR SEQ ID NO:17:

(i) SEQUENCE CHARACTERISTICS:
    (A) LENGTH: 20 amino acids
    (B) TYPE: amino acid
    (C) STRANDEDNESS: single

(D) TOPOLOGY: unknown

(ii) MOLECULE TYPE: peptide

(iii) HYPOTHETICAL: NO

(iv) ANTI-SENSE: NO

(vi) ORIGINAL SOURCE:
    (C) INDIVIDUAL ISOLATE: B antigen, CSP peptide

(xi) SEQUENCE DESCRIPTION: SEQ ID NO:17:

Thr Cys Gly Val Gly Val Arg Val Arg Ser Arg Val Asn Ala Ala Asn
1           5             10           15

Lys Lys Pro Glu
        20

(2) INFORMATION FOR SEQ ID NO:18:

    (i) SEQUENCE CHARACTERISTICS:
        (A) LENGTH: 17 amino acids
        (B) TYPE: amino acid
        (C) STRANDEDNESS: single
        (D) TOPOLOGY: unknown

    (ii) MOLECULE TYPE: peptide

    (iii) HYPOTHETICAL: NO

    (iv) ANTI-SENSE: NO

    (vi) ORIGINAL SOURCE:
        (C) INDIVIDUAL ISOLATE: PAK peptide

    (xi) SEQUENCE DESCRIPTION: SEQ ID NO:18:

Lys Cys Thr Ser Asp Gln Asp Glu Gln Phe Ile Pro Lys Gly Cys Ser
1           5             10           15

Lys

(2) INFORMATION FOR SEQ ID NO:19:

   (i) SEQUENCE CHARACTERISTICS:
      (A) LENGTH: 105 base pairs
      (B) TYPE: nucleic acid
      (C) STRANDEDNESS: double
      (D) TOPOLOGY: both

   (ii) MOLECULE TYPE: DNA

   (iii) HYPOTHETICAL: NO

   (iv) ANTI-SENSE: NO

   (vi) ORIGINAL SOURCE:
      (C) INDIVIDUAL ISOLATE: E-coil sequence

   (ix) FEATURE:
      (A) NAME/KEY: CDS
      (B) LOCATION: 1..105

   (xi) SEQUENCE DESCRIPTION: SEQ ID NO:19:

```
GAG GTA TCC GCT TTA GAG AAA GAA GTT TCT GCT CTC GAA AAA GAG GTC        48
Glu Val Ser Ala Leu Glu Lys Glu Val Ser Ala Leu Glu Lys Glu Val
 1               5                   10                  15

AGT GCT CTG GAA AAA GAG GTG TCA GCC TTG GAA AAG GAA GTA TCA GCA        96
Ser Ala Leu Glu Lys Glu Val Ser Ala Leu Glu Lys Glu Val Ser Ala
            20                  25                  30

CTT GAG AAG                                                          105
Leu Glu Lys
            35
```

(2) INFORMATION FOR SEQ ID NO:20:

   (i) SEQUENCE CHARACTERISTICS:
      (A) LENGTH: 35 amino acids
      (B) TYPE: amino acid
      (D) TOPOLOGY: linear

   (ii) MOLECULE TYPE: protein

(xi) SEQUENCE DESCRIPTION: SEQ ID NO:20:

Glu Val Ser Ala Leu Glu Lys Glu Val Ser Ala Leu Glu Lys Glu Val
1               5               10              15

Ser Ala Leu Glu Lys Glu Val Ser Ala Leu Glu Lys Glu Val Ser Ala
            20              25              30

Leu Glu Lys
        35


(2) INFORMATION FOR SEQ ID NO:21:

    (i) SEQUENCE CHARACTERISTICS:
        (A) LENGTH: 105 base pairs
        (B) TYPE: nucleic acid
        (C) STRANDEDNESS: double
        (D) TOPOLOGY: both

    (ii) MOLECULE TYPE: DNA

    (iii) HYPOTHETICAL: NO

    (iv) ANTI-SENSE: NO

    (vi) ORIGINAL SOURCE:
        (C) INDIVIDUAL ISOLATE: K-coil sequence

    (ix) FEATURE:
        (A) NAME/KEY: CDS
        (B) LOCATION: 1..105


    (xi) SEQUENCE DESCRIPTION: SEQ ID NO:21:

AAG GTA TCC GCT TTA AAA GAG AAA GTT TCT GCT CTG AAA GAA AAG GTC    48
Lys Val Ser Ala Leu Lys Glu Lys Val Ser Ala Leu Lys Glu Lys Val
1               5               10              15

AGT GCT CTG AAG GAG AAG GTG TCA GCC TTG AAG GAA AAG GTT TCA GCA    96
Ser Ala Leu Lys Glu Lys Val Ser Ala Leu Lys Glu Lys Val Ser Ala
            20              25              30

```
CTT AAA GAG                                                          105
Leu Lys Glu
         35
```

(2) INFORMATION FOR SEQ ID NO:22:

     (i) SEQUENCE CHARACTERISTICS:
        (A) LENGTH: 35 amino acids
        (B) TYPE: amino acid
        (D) TOPOLOGY: linear

    (ii) MOLECULE TYPE: protein

    (xi) SEQUENCE DESCRIPTION: SEQ ID NO:22:

```
Lys Val Ser Ala Leu Lys Glu Lys Val Ser Ala Leu Lys Glu Lys Val
 1               5                  10                  15

Ser Ala Leu Lys Glu Lys Val Ser Ala Leu Lys Glu Lys Val Ser Ala
            20                  25                  30

Leu Lys Glu
       35
```

(2) INFORMATION FOR SEQ ID NO:23:

     (i) SEQUENCE CHARACTERISTICS:
        (A) LENGTH: 231 base pairs
        (B) TYPE: nucleic acid
        (C) STRANDEDNESS: double
        (D) TOPOLOGY: both

    (ii) MOLECULE TYPE: DNA

   (iii) HYPOTHETICAL: NO

    (iv) ANTI-SENSE: NO

    (vi) ORIGINAL SOURCE:
        (C) INDIVIDUAL ISOLATE: fragment in Fig. 13

    (ix) FEATURE:
        (A) NAME/KEY: CDS

(B) LOCATION: 1..219

(xi) SEQUENCE DESCRIPTION: SEQ ID NO:23:

```
CGA GAA TTC AAG TGT ACT TCT GAC CAA GAC GAG CAA TTC ATC CCT AAG          48
Arg Glu Phe Lys Cys Thr Ser Asp Gln Asp Glu Gln Phe Ile Pro Lys
 1               5                   10                  15

GGT TGT TCC AAA TTC GGA GGA GGT GGA GGT GGT GGT GGC GAG GTA TCC          96
Gly Cys Ser Lys Phe Gly Gly Gly Gly Gly Gly Gly Gly Glu Val Ser
                20                  25                  30

GCT TTA GAG AAA GAA GTT TCT GCT CTC GAA AAA GAG GTC AGT GCT CTG         144
Ala Leu Glu Lys Glu Val Ser Ala Leu Glu Lys Glu Val Ser Ala Leu
            35                  40                  45

GAA AAA GAG GTG TCA GCC TTG GAA AAG GAA GTA TCA GCA CTT GAG AAG         192
Glu Lys Glu Val Ser Ala Leu Glu Lys Glu Val Ser Ala Leu Glu Lys
         50                  55                  60

GGC GGT GGA GGA CAT CAC CAC CAT CAC TAATAAGGAT CC                       231
Gly Gly Gly Gly His His His His His
 65                  70
```

(2) INFORMATION FOR SEQ ID NO:24:

    (i) SEQUENCE CHARACTERISTICS:
        (A) LENGTH: 73 amino acids
        (B) TYPE: amino acid
        (D) TOPOLOGY: linear

    (ii) MOLECULE TYPE: protein

    (xi) SEQUENCE DESCRIPTION: SEQ ID NO:24:

```
Arg Glu Phe Lys Cys Thr Ser Asp Gln Asp Glu Gln Phe Ile Pro Lys
 1               5                   10                  15

Gly Cys Ser Lys Phe Gly Gly Gly Gly Gly Gly Gly Gly Glu Val Ser
                20                  25                  30

Ala Leu Glu Lys Glu Val Ser Ala Leu Glu Lys Glu Val Ser Ala Leu
            35                  40                  45
```

Glu Lys Glu Val Ser Ala Leu Glu Lys Glu Val Ser Ala Leu Glu Lys
50                    55                    60

Gly Gly Gly Gly His His His His His
65                    70

(2) INFORMATION FOR SEQ ID NO:25:

    (i) SEQUENCE CHARACTERISTICS:
        (A) LENGTH: 51 base pairs
        (B) TYPE: nucleic acid
        (C) STRANDEDNESS: double
        (D) TOPOLOGY: both

    (ii) MOLECULE TYPE: DNA

   (iii) HYPOTHETICAL: NO

    (iv) ANTI-SENSE: NO

    (vi) ORIGINAL SOURCE:
        (C) INDIVIDUAL ISOLATE: PAK antigen

    (ix) FEATURE:
        (A) NAME/KEY: CDS
        (B) LOCATION: 1..51

    (xi) SEQUENCE DESCRIPTION: SEQ ID NO:25:

AAG TGT ACT TCT GAC CAA GAC GAG CAA TTC ATC CCT AAG GGT TGT TCC          48
Lys Cys Thr Ser Asp Gln Asp Glu Gln Phe Ile Pro Lys Gly Cys Ser
1                   5                   10                  15

AAA                                                                     51
Lys

(2) INFORMATION FOR SEQ ID NO:26:

    (i) SEQUENCE CHARACTERISTICS:
        (A) LENGTH: 17 amino acids
        (B) TYPE: amino acid

(D) TOPOLOGY: linear

(ii) MOLECULE TYPE: protein

(xi) SEQUENCE DESCRIPTION: SEQ ID NO:26:

```
Lys Cys Thr Ser Asp Gln Asp Glu Gln Phe Ile Pro Lys Gly Cys Ser
 1               5                  10                  15
```

Lys

(2) INFORMATION FOR SEQ ID NO:27:

    (i) SEQUENCE CHARACTERISTICS:
        (A) LENGTH: 228 base pairs
        (B) TYPE: nucleic acid
        (C) STRANDEDNESS: double
        (D) TOPOLOGY: both

    (ii) MOLECULE TYPE: DNA

   (iii) HYPOTHETICAL: NO

    (iv) ANTI-SENSE: NO

    (vi) ORIGINAL SOURCE:
        (C) INDIVIDUAL ISOLATE: fragment in Fig. 14

    (ix) FEATURE:
        (A) NAME/KEY: CDS
        (B) LOCATION: 1..216

    (xi) SEQUENCE DESCRIPTION: SEQ ID NO:27:

```
CGA GAA TTC TTG TCT GAG ATC AAG GGA GTA ATC GTC CAC AGA CTT GAA      48
Arg Glu Phe Leu Ser Glu Ile Lys Gly Val Ile Val His Arg Leu Glu
 1               5                  10                  15

GGT GTC AAA TTC GGA GGA GGT GGA GGT GGT GGT GGC GAG GTA TCC GCT      96
Gly Val Lys Phe Gly Gly Gly Gly Gly Gly Gly Gly Glu Val Ser Ala
                20                  25                  30
```

```
TTA GAG AAA GAA GTT TCT GCT CTC GAA AAA GAG GTC AGT GCT CTG GAA          144
Leu Glu Lys Glu Val Ser Ala Leu Glu Lys Glu Val Ser Ala Leu Glu
        35                  40                  45


AAA GAG GTG TCA GCC TTG GAA AAG GAA GTA TCA GCA CTT GAG AAG GGC          192
Lys Glu Val Ser Ala Leu Glu Lys Glu Val Ser Ala Leu Glu Lys Gly
        50                  55                  60


GGT GGA GGA CAT CAC CAC CAT CAC TAATAAGGAT CC                           228
Gly Gly Gly His His His His His
    65                  70
```

(2) INFORMATION FOR SEQ ID NO:28:

    (i) SEQUENCE CHARACTERISTICS:
        (A) LENGTH: 72 amino acids
        (B) TYPE: amino acid
        (D) TOPOLOGY: linear

    (ii) MOLECULE TYPE: protein

    (xi) SEQUENCE DESCRIPTION: SEQ ID NO:28:

```
Arg Glu Phe Leu Ser Glu Ile Lys Gly Val Ile Val His Arg Leu Glu
 1               5                  10                  15

Gly Val Lys Phe Gly Gly Gly Gly Gly Gly Gly Glu Val Ser Ala
            20                  25                  30

Leu Glu Lys Glu Val Ser Ala Leu Glu Lys Glu Val Ser Ala Leu Glu
        35                  40                  45

Lys Glu Val Ser Ala Leu Glu Lys Glu Val Ser Ala Leu Glu Lys Gly
        50                  55                  60

Gly Gly Gly His His His His His
    65                  70
```

(2) INFORMATION FOR SEQ ID NO:29:

    (i) SEQUENCE CHARACTERISTICS:

(A) LENGTH: 45 base pairs

(B) TYPE: nucleic acid

(C) STRANDEDNESS: double

(D) TOPOLOGY: both


(ii) MOLECULE TYPE: DNA


(iii) HYPOTHETICAL: NO


(iv) ANTI-SENSE: NO


(vi) ORIGINAL SOURCE:

(C) INDIVIDUAL ISOLATE: MVF antigen


(ix) FEATURE:

(A) NAME/KEY: CDS

(B) LOCATION: 1..45


(xi) SEQUENCE DESCRIPTION: SEQ ID NO:29:


```
TTG TCT GAG ATC AAG GGA GTA ATC GTC CAC AGA CTT GAA GGT GTC                45
Leu Ser Glu Ile Lys Gly Val Ile Val His Arg Leu Glu Gly Val
 1               5                   10                  15
```


(2) INFORMATION FOR SEQ ID NO:30:


(i) SEQUENCE CHARACTERISTICS:

(A) LENGTH: 15 amino acids

(B) TYPE: amino acid

(D) TOPOLOGY: linear


(ii) MOLECULE TYPE: protein


(xi) SEQUENCE DESCRIPTION: SEQ ID NO:30:


```
Leu Ser Glu Ile Lys Gly Val Ile Val His Arg Leu Glu Gly Val
 1               5                   10                  15
```